# EUROPEAN PATENT SPECIFICATION

(11) **EP 4 371 471 B1**
(45) Date of publication and mention of the grant of the patent: **02.07.2025**
(21) Application number: 23205166.4
(22) Date of filing: 23.10.2023
(51) Int. Cl.: A61B 5/00

(54) **METHOD OF MEASURING A FLUORESCENCE SIGNAL AND A VISIBLE LIGHT IMAGE, IMAGE CAPTURING AND PROCESSING DEVICE**
VERFAHREN ZUR MESSUNG EINES FLUORESZENZSIGNALS UND EINES BILDES IM SICHTBAREN LICHT, BILDERFASSUNGS- UND VERARBEITUNGSVORRICHTUNG
PROCÉDÉ DE MESURE D'UN SIGNAL DE FLUORESCENCE ET D'UNE IMAGE DE LUMIÈRE VISIBLE, DISPOSITIF DE CAPTURE ET DE TRAITEMENT D'IMAGE

(30) Priority: 15.11.2022 US 202263425393 P
(43) Date of publication of application: 22.05.2024
(73) Proprietor: Quest Photonic Devices B.V., 1771 SR Wieringerwerf (NL)
(72) Inventor: KOOPMAN, Thomas, 1013 WR Amsterdam (NL); HOVELING, Richelle Johanna Maria, 1623 JA Hoorn (NL); SOEBRATA, Ferran, 1616 XA Hoogkarspel (NL); WELTEVREDEN, Tyrique, 1784 PD Den Helder (NL)
(74) Representative: Seemann & Partner Patentanwälte mbB

(56) References cited:
- WO-A1-2021/035094
- US-A1- 2015 148 630
- US-A1- 2015 198 797
- US-A1- 2022 322 922

## Description

The invention is defined in the appended claims. The present disclosure relates to a method of measuring a fluorescence signal in a tissue of a body part, to which a fluorescent agent has been added, and of imaging a surface of the body part, wherein the tissue to which the fluorescent agent has been added forms part of the body part. Furthermore, the disclosure relates to an image capturing and processing device configured to measure a fluorescence signal in a tissue of a body part, to which a fluorescent agent has been added and configured to image a surface of the body part, wherein the tissue to which the fluorescent agent has been added forms part of the body part. The disclosure also relates to an endoscope or laparoscope comprising such image capturing and processing device.

Furthermore, the disclosure relates to a method of diagnosing lymphedema and to a method of long-term therapy of lymphedema.

The method and the image capturing and processing device in particular relate to imaging and measuring of the lymphatic function, in particular in view of the diagnosis, treatment and/or prevention of lymphedema.

Lymphedema is an accumulation of lymphatic fluid in the body's tissue. While oxygenated blood is pumped via the arteries from the heart to the tissue, deoxygenated blood returns to the heart via the veins. Because the pressure level on the arterial side is much higher than on the vein side, a colorless fluid part of the blood is pushed into the space between the cells. Typically, more fluid is pushed out, than reabsorbed on the vein side. The excess fluid is transported by the lymphatic capillaries. Furthermore, the fluid carries away local and foreign substances such as larger proteins and cellular debris. Once in the lymphatic system, this fluid including the transported substances is referred to as lymph or lymph fluid.

The lymphatic system comprises lymphatic vessels having one way valves similar to vein valves for transporting the lymph to the next lymph node. The lymph node performs removal of certain substances and cleans the fluid before it drains back to the blood stream.

If the lymphatic system becomes obstructed in that the lymph flow is blocked or not performed at the desired level, the lymph fluid accumulates in the interstitial space between the tissue cells. This accumulation, which is due to an impairment of lymphatic transport, is called lymphedema. The accumulation of lymph can cause inflammatory reaction which damages the cells surrounding the affected areas. It can further cause fibrosis which can turn into a hardening of the affected tissue.

Because lymphedema is a lifelong condition for that no cure or medication exists, early diagnoses and appropriate early counter measures for improving drainage and reducing the fluid load are of high importance for patients' well-being and recovering. Possible treatments such as lymphatic massage and compression bandages up to surgery depend on the level of severity, which is a four stage system defined by the World Health Organization (WHO) as follows:

| | |
|---|---|
| Stage 1: | a normal flow in the lymphatic system. No signs or symptoms. |
| Stage 2: | accumulation of fluid with swelling. |
| Stage 3: | permanent swelling that does not resolve with elevation of the affected limb or body part. |
| Stage 4: | elephantiasis (large deformed limb), skin thickening with "wart like" growth and extensive scarring. |

For diagnosis of the function of the lymphatic system, commonly used techniques are a manual inspection of the affected limb or body part by a physician. A known imaging technique is lymphoscintigraphy. In this technique, a radio tracer is injected in the tissue of the affected body part and subsequently MRI (Magnetic Resonance Imaging), CT (Computer Tomography), a PET-CT-scan (Positron Emission Tomography) or ultrasound imaging is performed.

A relatively new imaging technique is infrared fluorescence imaging using a fluorescent dye, for example ICG (Indocyanine Green). ICG is a green colored medical dye that is used for over 40 years. The dye emits fluorescent light when exited with near infrared light having a wavelength between 600 nm and 800 nm. Due to this excitation, ICG emits fluorescence light between 750 nm and 950 nm. The fluorescence of the ICG dye can be detected using a CCD or CMOS sensor or camera. The fluorescent dye is administered to the tissue of an affected limb or body part and the concentration and flow of the lymphatic fluid can be traced on the basis of the detected fluorescence light.

US 2022/0322922 A1 and US 2015/0148630 A1 disclose methods and apparatuses for acquisition of images in the visible light range and fluorescence images in the infrared band. WO 2021/035094 A1 discloses that a stitching algorithm can be applied to merge individual images into a common and larger image. US 2015/198797 A1 discloses both of the above.

It is an object of the disclosure to provide an enhanced method of measuring a fluorescence signal and an enhanced image capturing and processing device as well as an enhanced endoscope or laparoscope, wherein in particular an enhanced fluorescence imaging output can be provided. The invention does not encompass capturing images by manipulating an image capturing device within a living body.

Furthermore, it is an object of the disclosure to provide an enhanced method of diagnosing lymphedema and an enhanced method of long-term therapy of lymphedema.

The object is solved by a method of measuring a fluorescence signal in a tissue of a body part, to which a fluorescent agent has been added, and of imaging a surface of the body part, wherein the tissue to which the fluorescent agent has been added forms part of the body part, the method comprising the steps of:
capturing a fluorescence image by illuminating the tissue with excitation light having a wavelength suitable to generate emitted light by excited emission of the fluorescent agent, and by spatially resolved measurement of the emitted light so as to provide the fluorescence image,
capturing a visible light image of at least a section of a surface of the body part, wherein a viewing direction and/or a perspective of the fluorescence image and the visible light image are linked via a known relationship and
the method further comprises the steps of:
repeating the capturing of the fluorescence image and the visible light image to provide a series of fluorescence images and a series of visible light images, wherein
the step of capturing the series of fluorescence images and the series of visible light images does not encompass capturing of images by manipulating an endoscope within a living body comprising the body part, during a surgical procedure performed on the body,
applying a stitching algorithm on the series of visible light images to generate a large visible light image of the body part, wherein the stitching algorithm determines and applies a set of stitching parameters,
applying the stitching algorithm on the series of fluorescence images to generate a large fluorescence image, wherein the stitching algorithm applies the set of stitching parameters determined when performing the stitching of the visible light images, if the viewing direction and perspective of the visible light images and the fluorescence images are identical, and the stitching algorithm applies the set of stitching parameters having a fixed offset, if the viewing direction and perspective of the visible light images and the fluorescence images are not identical, the offset reflecting the known relationship linking the viewing direction and perspective of the fluorescence image and the visible light image,
outputting the large visible light image and the large fluorescence image.

Advantageously, two images, namely the large visible light image and the large fluorescent image, are output, wherein in contrast to traditional methods these two images are linked to each other. In other words, the two images share a common coordinate system, which means that objects that can be seen in one of the images, for example in the fluorescence image, can be found in the visible light image at the same position. The visible light image reproduces the surface of the body part as it can be objected with the human eye. In the fluorescence image, an intensity of the fluorescence light is reproduced. In particular, both images are displayed using the same image scale, image orientation and show the same region of interest of the body part. This is due to the fact, both images are captured with in particular identical viewing direction and identical field of view. Based on the image information that is provided, the user is set into position to exactly spot the point or position at which a certain fluorescence phenomena is observed, at the real body part. This provides unparalleled advantages with respect to diagnosis and therapy.

Within the context of this specification the terms "fluorescence image" and "visible light image" are not limited to 2D images. Images within the meaning of this specification can be 2D images but also 3D images or 2D images comprising additional information. 2D images can be captured using a specialized camera equipment. 3D images can be captured using for example a stereo camera equipment. The 2D or 3D images can also be line scans, which can be captured using an image line scanner. The images can also be LIDAR scans that similar to 3D images also comprise a depth information. The data from the LIDAR scanner can be added to 2D images as additional information. The combination of the 2D image data and the LIDAR scan data leads to the similar information as a 3D image because the 2D image data can be combined with corresponding depth information. Furthermore, the term "stitching" is not limited to the combination of two or more 2D images. Stitching can also be performed on the basis of the other above mentioned image data, for example on the basis of 3D image data. Further information like for example the depth information that is captured by the LIDAR scanner can also be taken into account when executing the stitching algorithm.

Within the context of this specification, a "visible light image" is an image of the real world situation. It reproduces an image impression similar to what can be seen by the human eye. Unlike the human eye, the visible light image can be a color image, a greyscale image or even a false color scale plot. The visible light image shows the surface of the body part comprising the tissue to which the fluorescent agent has be administered. If the tissue is arranged at the surface of the body part, the imaging of the surface of the body part includes imaging of the surface of the tissue.

Stitching can be performed on the basis of two or more 2D images and results in a larger 2D image. When the stitching algorithm is executed on the basis of a plurality of 3D images, the result is a large 3D image. It is also possible to perform the step of stitching in that a plurality of 2D images plus additional information (for example from a LIDAR scanner, a time of flight sensor or a similar device) is processed and the result of this is a large 3D image. In this case, the step of stitching includes the reconstruction of a 3D image from a 2D image data plus additional information. Stitching of images comprises the step of identifying unique special features that correspond to each other and are shown in the two images that have to be stitched together. This requires that the field of view of the two images that are combined in the stitching process overlap at least slightly.

In view of this prerequisite for the stitching process, according to an embodiment of the disclosure, the step of repeating the capturing of the fluorescence image and the visible light image to provide a series of fluorescence images and a series of visible light images, is executed in that the individual subsequent images of said sequence are captured in that the field of view of the subsequent images are at least slightly overlapping.

The previously mentioned additional information, which can be used for the stitching process, is however not limited to depth information, which is assigned to for example every individual pixel in the 2D image. It is also possible that during the image acquisition of the series of fluorescent images and visible light images, the image capturing device acquires, as further data, data about spatial orientation of the image capturing device. The spatial orientation can be for example an orientation of the image acquisition device in the coordinate system of an examination room. This orientation can be characterized by the three space coordinates x, y and z together with a viewing direction (for example a vector in the coordinate system of the examination room) and a tilt angle of the image capturing device about the viewing direction (for example an angle of rotation of the image capturing device about the viewing direction). This information can be captured for every single image (or image pair comprising a fluorescence image and a visible light image) of the series of visible light images and fluorescence images. When performing the step of stitching, this additional information indicating the orientation of the image capturing device in space can be used. In particular, the orientation of the image capturing device in for example a coordinate system of the examination room can be recalculated into an orientation of the image capturing in relation to the body part. The information can be used when performing the 3D reconstruction of the body part during the step of stitching.

Furthermore, the stitching algorithm can also be applied in any other application to generate a large visible light image and a large fluorescence image. In other words, the method is in particular not limited to the assessment of the lymphatic system. The assessment can be performed on for example blood vessels and blood flow or on a perfusion assessment of organs and tissues. The assessment can also encompass visually locating tissue with certain characteristica (e.g. tumorous tissue), locating glands (e.g. parathyroid glands) and nerves. This list is not exhaustive.

The stitching algorithm can be applied on images obtained in open surgery or in minimally invasive sugery. Images on which stitching is performed and which are captured during minimally invasive sugery, can be obtained using an endoscope, for example an endoscope having a rigid shaft or an endoscope having a flexible shaft.

According to these further aspects of the disclosure, there is a method of measuring a fluorescence signal in a body part, to which a fluorescent agent has been added, and of imaging a surface of the body part, the method comprising the steps of:
capturing a fluorescence image by illuminating the body part with excitation light having a wavelength suitable to generate emitted light by excited emission of the fluorescent agent, and by spatially resolved measurement of the emitted light so as to provide the fluorescence image,
capturing a visible light image of at least a section of a surface of the body part, wherein a viewing direction and/or a perspective of the fluorescence image and the visible light image are linked via a known relationship,
repeating the capturing of the fluorescence image and the visible light image to provide a series of fluorescence images and a series of visible light images,
applying a stitching algorithm on the series of visible light images to generate a large visible light image of the body part, wherein the stitching algorithm determines and applies a set of stitching parameters,
applying the stitching algorithm on the series of fluorescence images to generate a large fluorescence image, wherein the stitching algorithm applies the set of stitching parameters determined when performing the stitching of the visible light images.

In particular, the method further comprises the step of:
outputting the large visible light image and the large fluorescence image.

The method is in particular not limited to the use of a fluorescent agent and/or dye. The method can be performed without the use of a dye, by exploiting the effect of auto-fluorescence of certain tissue, for example of parathyroid glands or intestinal tissue. Furthermore, the absence of auto-fluorescence can be used for example to determine lesions.

According to this aspect of the disclosure, a method of measuring an auto-fluorescence signal in a tissue of a body part or in a body part is provided. It is not necessary to add or administer a fluorescent agent to the tissue or to the body part. The method comprises the step of imaging a surface of the body part, wherein the tissue in which auto-fluorescence if measured forms part of the body part. The method further comprising the steps of:
capturing an auto-fluorescence image by illuminating the tissue or the body party with excitation light having a wavelength suitable to generate emitted light by excited auto-fluorescence emission of the tissue or the body part, and by spatially resolved measurement of the emitted light so as to provide the auto-fluorescence image,
capturing a visible light image of at least a section of a surface of the body part, wherein a viewing direction and/or a perspective of the auto-fluorescence image and the visible light image are linked via a known relationship,
repeating the capturing of the auto-fluorescence image and the visible light image to provide a series of auto-fluorescence images and a series of visible light images,
applying a stitching algorithm on the series of visible light images to generate a large visible light image of the body part, wherein the stitching algorithm determines and applies a set of stitching parameters,
applying the stitching algorithm on the series of auto-fluorescence images to generate a large auto-fluorescence image, wherein the stitching algorithm applies the set of stitching parameters determined when performing the stitching of the visible light images.

The method in particular comprises the step of outputting the large visible light image and the large auto-fluorescence image.

According to still another aspect of the disclosure, the method comprises the steps of:
applying a stitching algorithm on the series of fluorescence or auto-fluorescence images to generate a large fluorescence image or a large auto-fluorescence image, wherein the stitching algorithm determines and applies a set of stitching parameters,
applying the stitching algorithm on the series of visible light images to generate a large visible light image, wherein the stitching algorithm applies the set of stitching parameters determined when performing the stitching of the fluorescence or auto-fluorescence images.

In other words, it is possible to apply the set of stitching parameters, which have been determined when stitching the visible light images, on the fluorescence images and vice versa.

Furthermore, it is within the scope of above explained method that the fluorescence image is in fact an image detected in the visible light spectrum. This applies for example, if Patent Blue, methylene blue or isosulfan blue is applied as the fluorescent agent or dye, because the light emission of this substance can be seen with the naked eye.

According to an advantageous embodiment of the disclosure, the method further comprises the steps of superimposing the large visible light image and the large fluorescence image to provide an overlay image of the body part and outputting the overlay image as output of the large visible light image and the large fluorescence image.

Advantageously, the method according to aspects of the disclosure provides an overlay image showing the visible light image of the body part, which is for example affected by lymphedema, and the corresponding fluorescence image, which is indicative of a concentration of lymph in the respective region of the affected body part. In other words, the overlay image is a combination of a visible light image and a fluorescence image. The fluorescence image can be for example an image, in which the intensity of the fluorescence light is shown as a false color plot. The overlay image enhances and simplifies the analysis of the situation of the lymphatic system. In traditional measurement methods for detecting a fluorescence signal, there is typically no visible light image combined with the fluorescence image. This, however, makes it very difficult to pinpoint exact locations, at which abnormalities in the lymphatic transport are detected, because they are for example visible as intensity spots in the fluorescence image. This drawback becomes even more relevant if different measurements are performed at different points in time or by different operators. Without the link to the visible light image, it is very difficult to compare fluorescence measurements, which have been taken at different points in time or by different persons. If no visible light image is combined with the fluorescence image or linked thereto, it is difficult to perform patient follow up or to monitor any progress of a therapy.

The step of stitching the fluorescence images advantageously includes the reconstruction of the images to a 3D image. Stitching of the series of fluorescence images is performed based on the set of stitching parameters, which have been previously determined when performing the stitching of the visible light images.

Fluorescence images typically offer rare special features that are suitable for performance of the stitching process. Because the visible light image and the fluorescence image are linked by a known and constant relationship with respect to viewing direction and/or perspective, the parameters of the stitching algorithm used for the visible light images can also be applied for the stitching of the fluorescence images. In other words, due to the fact the visible light image and the fluorescence image are captured via optically aligned sensors, the same stitching parameters can be used for stitching of the visible light images and for stitching of the fluorescence images. This significantly enhances the stitching process and results in a large fluorescence image of better quality.

The fluorescent agent is for example ICG (Indocyanine Green) or methylene blue. Within the context of this specification, the term "fluorescent dye" or "dye" (also referred to as "fluorochrome" or "fluorophore") refers to a component of a molecule, which causes the molecule to be fluorescent. The component is a functional group in the molecule that absorbs energy of a specific wavelength and re-emits energy at a different specific wavelength. In various aspects, the fluorescent agent comprises a fluorescence dye, an analogue thereof, a derivative thereof, or a combination of these. Appropriate fluorescent dyes include, but are not limited to, indocyanine green (ICG), fluorescein, methylene blue, isosulfan blue, Patent Blue, cyanine5 (Cy5), cyanine5.5 (Cy5.5), cyanine7 (Cy7), cyanine7.5 (Cy7.5), cy-pate, silicon rhodamine, 5-ALA, IRDye 700, IRDye 800CW, IRDye 800RS, IRDye 800BK, porphyrin derivatives, Illuminare-1, ALM-488, GCP-002, GCP-003, LUM-015, EMI-137, SGM-101, ASP-1929, AVB-620, OTL-38, VGT-309, BLZ-100, ONM-100, BEVA800.

In embodiments where fluorescence is derived from autofluorescence, one or more of the fluorophores or agents giving rise to the autofluorescence may be an endogenous tissue fluorophore (e.g., NADH, thyroid gland, parathyroid gland etc.).

The method according to aspects of the disclosure can be performed with different fluorescent agents and dyes. Basically, any combination of an appropriate dye plus the suitable or matching excitation light source can be applied. The method can also be performed without the use of a dye exploiting the effect of auto-fluorescence of certain tissue (e.g. parathyroid glands) or molecules.

Examples for dyes that can be applied and are mainly used for the lymphatic system are for example: Indocyanine Green (ICG), methylene blue, isosulfan blue or Patent Blue. Patent Blue can even be seen with the naked eye. Hence, no fluorescence is needed, but the stitching of the images can be performed anyway.

The stitching algorithm is for example a panorama stitching algorithm, in which the images are analyzed to extract special and distinguishing features. These features are then linked to each other in the multiple images and image transformation (for example shifting, rotation, stretching along one or more axis or a keystone correction) is performed. The location of the linked features is used to determine the image transformation parameters, which are also referred to as the stitching parameters. Subsequent to image orientation, the images are merged and thereby "stitched" together. This transformation can be executed in the same way on both, the visible light images and the fluorescence images. Finally, the two images are output. This output can include the step of displaying the images for example on a screen. For example the two images are shown side-by-side. According to the above referred embodiment the two images are shown in an overlay image.

As previously mentioned, the step of performing the stitching of the series of images is not limited to the stitching of 2D images. The step of stitching can also comprise a reconstruction of a 3D image based on for example a series of 3D images or based on a series of 2D images plus additional information.

Often, the function of the lymphatic system is affected in a limb of the body. However, the method of measuring the fluorescence signal is not limited to the inspection of a limb. The method generally refers to the inspection of a body part, which can be a limb of a patient but also the corpus, the head, the neck, the back or any other part of the body. It is also possible that the body part is an organ. The method of measuring the fluorescence signal as an indicator for the lymphatic flow is in this case performed during open surgery. The same applies to a situation in which the surgery is minimally invasive surgery, which is performed using an endoscope or laparoscope.

According to another advantageous embodiment, the viewing direction and the perspective of the fluorescence image and the visible light image are identical and, in particular, the fluorescence image and the visible light image are captured through one and the same, in particular through one single, objective lens.

Advantageously, the fluorescence image and the visible light image can be captured by an image capturing device, which comprises a prism assembly and a plurality of image sensors assigned thereto.

Fluorescent light and visible light enter the prism assembly as a common light bundle and through one and the same entrance surface of the prism assembly. The prism structure comprises filters for separating the visible wavelength range from the infrared wavelength range, at which the exited emission of the fluorescent agent typically takes place. The different wavelength bands, i.e. the visible light (also abbreviated Vis) and the infrared light (is also abbreviated IR), are directed to different sensors. The capturing of the visible light image and the fluorescence image through one single objective lens allows a perfect alignment of the viewing direction and perspective of the two images. In particular, the viewing direction and the perspective of the visible light image and the fluorescence image are identical.

In an advantageous embodiment, capturing of the fluorescence image and capturing of the visible light image are performed simultaneously in absence of time-switching between a signal of the fluorescence image and a signal of the visible light image.

Advantageously, the method dispenses with time-switching of signals. This is advantageous, because the infrared image, which is the fluorescence image, and the visible light image are captured exactly at the same time using separate image sensors. Hence, the images can also be captured with a high frame repeat rate, which allows the method to be applied in situations where reliable hand eye coordination is necessary. High frame rates of 60 fps or even higher are possible. High frame rates can typically not be achieved when time-switching is applied. Furthermore, when the fluorescence image and the visible light image are captured on individual sensors, the sensors can be arranged exactly in focus. Furthermore, the settings of the sensors can be adjusted to the individual requirements for image acquisition of the visible light image and fluorescence image. This pertains for example to an adjustment of the sensor gain, the noise reduction, the exposure time, etc..

According to yet another advantageous embodiment, the steps of capturing the fluorescence image, illuminating the tissue with excitation light and simultaneously capturing the visible light image are performed by a single image capturing device. When illumination and image acquisition are integrated in one device, the overall process of measurement of the fluorescence signal and simultaneous acquisition of visible images can be enhanced.

According to an advantageous aspect of this embodiment, the method further comprises the step of measuring a distance between a surface of the body part, which is captured in the visible light image and the capturing device and in particular outputting a signal by the image capturing device, which is indicative of the measured distance. Measurements at different distances can be performed to optimize the illumination and image capture to find the best image acquisition conditions. The distance of this best fit can then be stored in the imaging system as a target distance for following measurements.

According to an embodiment of the disclosure, the method can be further enhanced in that it comprises the step of outputting a signal by the image capturing device, which is indicative of the measured distance. For example, a visual signal and/or an audio signal can be output by the image capturing device. This signal can guide the operator when handling the image capturing device such that image acquisition is performed at an at least approximately constant distance to the surface of the body part. The integration of the distance sensor in the image capturing device and the user supporting output (visual or optical signal) enable the operator to capture images with more homogeneous illumination. This enhances the quality of the measurement of the fluorescence signal.

For determination of the best fit distance, the method can further comprise the steps of repeatedly capturing the fluorescence image and the visible light image of the same section of the surface of the body part while measuring the distance. A plurality of sets of fluorescence and visible light images are captured at different distances. Subsequently, an analysis of the sets of images in view of imaging quality is performed and a best matching distance resulting in the highest quality of images is determined.

The output signal, which can be an audio signal, an optical signal or can be generated based on a basis of a measurement of a time of flight sensor, can also be indicative of a deviation of the measured distance from the best matching distance. Hence, the operator is directly informed whether or not the optimum image capturing conditions in particular with respect to illumination are applied during the measurement.

Furthermore, the step of image capturing can be performed by a robot or another automatic camera holder. In this embodiment, the output signal is used as an input for the robot or the automatic camera holder to adjust to best matching distance. No signal output is necessary in this case but the robot or camera holder will automatically move according to a stored and best matching distance.

According to still another embodiment of the disclosure, the measurement of the fluorescence signal is performed on a tissue, to which at least a first and a second fluorescent agent has been added, wherein the step of capturing the fluorescence image comprises:
capturing a first fluorescence image in a first wavelength range, which is generated by illuminating the tissue with first excitation light having a first wavelength suitable to generate emitted light by a first excited emission of the first fluorescent agent, and
capturing a second fluorescence image in a second wavelength range, which is generated by illuminating the tissue with second excitation light having a second wavelength suitable to generate emitted light by a second excited emission of the second fluorescent agent, and the method further comprises the steps of:
   repeating the capturing of the first and the second fluorescence image to provide a first and a second series of fluorescence images,
   applying the stitching algorithm on the first and second series of fluorescence images to generate a first and a second large fluorescence image, wherein the stitching algorithm applies the set of stitching parameters determined when performing the stitching of the visible light images,
   outputting the first and the second large fluorescence image.

According to the above embodiment, the fluorescent agent comprises a first and a second fluorescent dye. The first fluorescent dye can be for example a methylene blue, the second dye can be ICG. The step of capturing the fluorescence image, according to this embodiment, comprises capturing a first fluorescence image of the fluorescent light emitted by the first fluorescent dye and, capturing a second fluorescence image of the fluorescent light emitted by the second fluorescent dye. Capturing of the two images is in particular performed without time switching. The first fluorescence image can be captured in a wavelength range, which is between 700 nm and 800 nm, if methylene blue is used as the first fluorescent dye. The second fluorescence image can be captured in a wavelength range, which is between 800 nm and 900 nm, if ICG is used as the second fluorescent dye. Fluorescence imaging which is based on two different fluorescent agents offers new possibilities for measurements and diagnosis.

The object is further solved by an image capturing and processing device configured to measure a fluorescence signal in a tissue of a body part, to which a fluorescent agent has been added, and configured to image a surface of the body part, wherein the tissue to which the fluorescent agent has been added forms part of the body part, the image capturing an processing device comprising an imaging unit, which further comprises:
an illumination unit configured to illuminate the tissue with excitation light having a wavelength suitable to generate emitted light by excited emission of the fluorescent agent,
a fluorescence imaging unit configured to capture a fluorescence image by spatially resolved measurement of the emitted light so as to provide a fluorescence image,
a visible light imaging unit configured to capture a visible light image of a section of a surface of the body part, wherein the fluorescence imaging unit and the visible light imaging unit are configured in that a viewing direction and/or a perspective of the fluorescence image and the visible light image are linked via a known relationship, wherein
the fluorescence imaging unit and the visible light imaging unit are further configured to repeat capturing of the fluorescence image and the visible light image to provide a series of fluorescence images and a series of visible light images, the image capturing an processing device comprising a processing unit, which further comprises
a stitching unit configured to apply a stitching algorithm on the series of visible light images to generate a large visible light image of the body part, the stitching algorithm determining and applying a set of stitching parameters,
wherein the stitching unit is further configured to apply the stitching algorithm on the series of fluorescence images to generate a large fluorescence image, wherein the stitching algorithm applies the set of stitching parameters determined when performing the stitching of the visible light images, if the viewing direction and perspective of the visible light images and the fluorescence images are identical, and the stitching unit is configured to apply the stitching algorithm in that it applies the set of stitching parameters having a fixed offset, if the viewing direction and perspective of the visible light images and the fluorescence images are not identical, the offset reflecting the known relationship linking the viewing direction and perspective of the fluorescence image and the visible light image,
an output unit configured to output the large visible light image and the large fluorescence image.

Same or similar advantages and advantageous aspects, which have been mentioned with respect to the method of measuring the fluorescence signal, apply to the image capturing and processing device in a same or similar way and will therefore not be repeated.

The device is configured in that the stitching unit is configured to apply the same stitching algorithm for stitching of the visible light images and for stitching of the fluorescence images. The stitching algorithm uses the same stitching parameters, which have been determined and used for the stitching of the visible images for the stitching of the fluorescence images. The device is also configured in that the stitching unit is configured to apply the stitching algorithm in that the same stitching parameters, which have been determined and used for the stitching of the fluorescence images, are applied for stitching of the visible light images. As previously mentioned, the stitching is not limited to the stitching of 2D images. It is also possible to perform stitching on the basis of 3D images. Furthermore, the stitching can include an image reconstruction of 3D images. This can also be performed by the stitching unit. The stitching unit can be further configured to take into account additional information for performance of for example a 3D reconstruction. This additional information can be for example an orientation of the image capturing device or a depth information, which forms part of the 3D image or is the result of a scanning procedure. According to an embodiment of the disclosure, the image capturing device comprises a scanner for scanning of a surface of the body part, for example a LIDAR scanner, a time of flight camera or any other comparable device.

The device is not limited to the assessment of the lymphatic system. The device can be configured for assessment of for example blood vessels and blood flow or on a perfusion assessment of organs and tissues. The assessment can also encompass visually locating tissue with certain characteristica (e.g. tumorous tissue), locating glands (e.g. parathyroid glands) and nerves. This list is not exhaustive.

Furthermore, device is not limited to the use of a fluorescent agent and/or dye. The device can be operated without the use of a dye, by exploiting the effect of auto-fluorescence of certain tissue, for example of parathyroid glands. Hence, according to further aspects of the disclosure, there is an image capturing and processing device configured to measure an auto-fluorescence signal in a tissue of a body part or on a body part, wherein the image capturing and processing device is further configured to image a surface of the body part, wherein the tissue forms part of the body part, the image capturing and processing device comprising an imaging unit, which further comprises:
an illumination unit configured to illuminate the tissue with excitation light having a wavelength suitable to generate emitted light by excited auto-emission,
a fluorescence imaging unit configured to capture an auto-fluorescence image by spatially resolved measurement of the emitted light so as to provide an auto-fluorescence image,
a visible light imaging unit configured to capture a visible light image of a section of a surface of the body part, wherein the fluorescence imaging unit and the visible light imaging unit are configured in that a viewing direction and/or a perspective of the auto-fluorescence image and the visible light image are linked via a known relationship, wherein
the fluorescence imaging unit and the visible light imaging unit are further configured to repeat capturing of the auto-fluorescence image and the visible light image to provide a series of auto-fluorescence images and a series of visible light images, the image capturing and processing device comprising a processing unit, which further comprises:
   a stitching unit configured to apply a stitching algorithm on the series of visible light images to generate a large visible light image of the body part, the stitching algorithm determining and applying a set of stitching parameters,
   wherein the stitching unit is further configured to apply the stitching algorithm on the series of auto-fluorescence images to generate a large auto-fluorescence image, wherein the stitching algorithm applies the set of stitching parameters determined when performing the stitching of the visible light images,
   an output unit configured to output the large visible light image and the large auto-fluorescence image.

According to another embodiment, the image capturing and processing device is further enhanced in that it further comprises a superimposing unit configured to superimpose the large visible light image and the large fluorescence image to provide an overlay image of the body part, wherein the output unit is further configured to output the overlay image as output of the large visible light image and the large fluorescence image.

According to yet another advantageous embodiment, the device is further enhanced in that the fluorescence imaging unit and the visible light imaging unit are configured in that the viewing direction and the perspective of the fluorescence image and the visible light image are identical, wherein in particular the fluorescence imaging unit and the visible light imaging unit are configured in that the fluorescence image and the visible light image are captured through one and the same objective lens.

Furthermore, the device is enhanced in that the fluorescence imaging unit and the visible light imaging unit are configured to capture the fluorescence image and the visible light image simultaneously, in absence of time-switching between a signal of the fluorescence image and a signal of the visible light image.

Furthermore, the image capturing device, which comprises the fluorescence imaging unit and the visible light imaging unit further comprises a dichroic prism assembly configured to receive fluorescent light and visible light through an entrance face, comprising: a first prism, a second prism, a first compensator prism located between the first prism and the second prism,
a further dichroic prism assembly for splitting the visible light in three light components, and a second compensator prism located between the second prism and the further dichroic prism assembly,
wherein the first prism and the second prism each have a cross section with at least five corners, each corner having an inside angle of at least 90 degrees, wherein the corners of the first prism and the second prism each have a respective entrance face and a respective exit face, and are each designed so that an incoming beam which enters the entrance face of the respective prism in a direction parallel to a normal of said entrance face is reflected twice inside the respective prism and exits the respective prism through its exit face parallel to a normal of said exit face,
wherein the normal of the entrance face and the normal of the exit face of the respective prism are perpendicular to each other; and
wherein, when light enters the first prism through the entrance face, the light is partially reflected towards the exit face of the first prism thereby traveling a first path length from the entrance face of the first prism to the exit face of the first prism, and the light partially enters the second prism via the first compensator prism and is partially reflected towards the exit face of the second prism, thereby traveling a second path length from the entrance face of the first prism to the exit face of the second prism,
wherein the first prism is larger than the second prism so that the first and the second path lengths are the same.

Advantageously, the above-referred five prism assembly allows to capture two fluorescence imaging wavelengths and the three colors for visible light imaging, for example red, blue and green. The five prism assembly is advantageous in that the optical paths of the light traveling from the entrance surface to a respective one of the sensors have identical length. Hence, all sensors are in focus and furthermore, there is no timing gap between the signals of the sensors. Advantageously, the device does not require time-switching of the received signals. This allows an image capture using a high frame rate and enhanced image quality.

According to still another aspect, the image capturing device, which comprises the fluorescence imaging unit and the visible light imaging unit, defines a first, a second, and a third optical path for directing fluorescence light and visible light to a first, a second, and a third sensor, respectively, the image capturing device further comprises a dichroic prism assembly, configured to receive the fluorescent light and the visible light through an entrance face, the dichroic prism assembly comprising: a first prism, a second prism and a third prism, each prism having a respective first, second, and third exit face, wherein: the first exit face is provided with the first sensor, the second exit face is provided with the second sensor, and the third exit face is provided with the third sensor, wherein in particular the first optical path is provided with a first filter, the second optical path is provided with a second filter, and the third optical path is provided with a third filter, wherein
the first, second, and third filters, in any order, are a green filter, an infrared filter, and a red/blue patterned filter comprising red and blue filters in alternating pattern so that half of the light received by the red/blue patterned filter goes through a blue filter and half of the light received by the red/blue patterned filter goes through a red filter.

Furthermore, according to another aspect, the first, second, and third filters, in any order, are a red/green/blue patterned filter (RGB filter), a first infrared filter, and a second infrared filter, wherein in particular, the first and second infrared filter have different transmission wavelength.

In other words, the first and second infrared filters are for filtering IR-light in different IR wavelength intervals, for example in a first IR-band in which typical fluorescent dyes emit a first fluorescence peak and in a second IR-band in which a typical fluorescent dye emits a second fluorescence peak. Typically, the second IR-band is located at higher wavelength compared to the first IR-band. The first and second infrared filter can also be adjusted to emission bands of different fluorescent agents. Hence, the emission of for example a first fluorescent agent passes the first filter (and is in particular blocked by the second filter) and can be detected on the corresponding first sensor and the emission of the second fluorescent agent passes the second filter (and is in particular blocked by the first filter) and can be detected on the corresponding second sensor. For example, the first filter can be configured to measure the fluorescence emission of methylene blue and the second filter can be configured to measure the fluorescence emission of ICG.

The image capturing and processing device can be also enhanced in that the illumination unit, the fluorescence imaging unit and the visible light imaging unit are arranged in a single image capturing device, which further comprises a measurement unit configured to measure a distance between the surface of the body part, which is captured in the visible light image, and in particular the image capturing device is configured to output a signal, which is indicative of the measured distance.

Also with respect to the embodiments, same or similar advantages and advantageous aspects apply, which have been mentioned with respect to the method of measuring the fluorescence signal.

The object is further solved by an endoscope or laparoscope being configured as the image capturing device in an image capturing and processing device according to one or more of the previously mentioned embodiments. Advantageously, the device for image capturing and processing can be used in open surgery as well as during surgery using an endoscope or laparoscope. Same or similar advantages, which have been mentioned with respect to the method and/or the device apply to the endoscope or laparoscope in a same or similar way.

The object is further solved by a method of diagnosing lymphedema, comprising the steps of:
administering a fluorescent agent to a body part,
measuring a fluorescence signal in a tissue of the body part, to which the fluorescent agent has been administered, and imaging a surface of the body part, wherein the tissue to which the fluorescent agent has been added forms part of the body part,
capturing a fluorescence image by illuminating the tissue with excitation light having a wavelength suitable to generate emitted light by excited emission of the fluorescent agent, and by spatially resolved measurement of the emitted light so as to provide the fluorescence image,
capturing a visible light image of at least a section of a surface of the body part, wherein
a viewing direction and/or a perspective of the fluorescence image and the visible light image are linked via a known relationship and
repeating the steps of capturing of the fluorescence image and the visible light image to provide a series of fluorescence images and a series of visible light images,
applying a stitching algorithm on the series of visible light images to generate a large visible light image of the body part, wherein the stitching algorithm determines and applies a set of stitching parameters,
applying the stitching algorithm on the series of fluorescence images to generate a large fluorescence image, wherein the stitching algorithm applies the set of stitching parameters determined when performing the stitching of the visible light images
outputting the large visible light image and the large fluorescence image,
deriving a diagnostic result relative to a severity of lymphedema by analyzing the output images.

The method of diagnosing lymphedema can be advantageously performed with higher precision and reliability and therefore provides better results. This entirely new approach can advantageously replace the classical way of diagnosing lymphedema. The traditional way to diagnose lymphedema is to perform a manual inspection of the affected body parts by a physician. This method of performing the diagnosis, however, inevitably includes a non-reproducible and random component, which is due to the individual experience and qualification of the physician. Furthermore, the method of diagnosing lymphedema includes same or similar advantages, which have been previously mentioned with respect to the method of measuring the fluorescent signal.

The method of diagnosing lymphedema is further enhanced in that the fluorescent agent is administered to an arm or leg of a patient by injecting the fluorescent agent in tissue between phalanges of the foot or hand of the patient. Arms and/or legs are typically affected by lymphedema. Hence, the application of a new and successful method of diagnosing lymphedema is particularly useful when performed with respect to these limbs.

The object is also solved by a method of long-term therapy of lymphedema, comprising the steps of diagnosing a severity of lymphedema by performing the steps according to the previously mentioned method of diagnosing lymphedema on a patient. Furthermore, the method of long-term therapy comprises the steps of
performing a therapy on the patient, the therapy being adjusted to the diagnostic result relative to the severity of lymphedema,
repeating the steps of diagnosing the severity of lymphedema and performing a therapy on the patient, wherein in each iteration, the therapy is adjusted to the detected severity of lymphedema.

The method of long-term therapy according to aspects of the invention is particularly useful because the diagnosis of lymphedema provides - in contrast to traditional methods - objective results with respect to the severity of the disease. The success of a long-term therapy can be analyzed from an objective point of view. The analysis and diagnosis is therefore much more valuable when looking at the success of the therapy.

Further characteristics of the disclosure will become apparent from the description of the embodiments according to the disclosure together with the claims, which define the invention, and the included drawings. Embodiments according to the disclosure can fulfill individual characteristics or a combination of several characteristics.

The disclosure is described below, based on exemplary embodiments, wherein reference is made expressly to the drawings with regard to the disclosure of all details according to the disclosure that are not explained in greater detail in the text. The drawings show in:
- Fig. 1: a schematic illustration of an image capturing and processing device,
- Fig. 2: a schematic illustration of an image capturing device and a processing unit of the imaging capturing and processing device,
- Fig. 3a): an example of a visible light image and
- Fig. 3b): the corresponding fluorescence image,
- Fig. 4: a large overlay image, which is in part generated from the visible light and fluorescence images shown in Figs. 3a) and 3b),
- Fig. 5: a schematic illustration showing an internal prism assembly of the image capturing device,
- Fig. 6: a schematic illustration of an endoscope or laparoscope including the image capturing device,
- Fig. 7: a flowchart of a stitching algorithm,
- Fig. 8: a schematic illustration showing another internal prism assembly of the image capturing device.

In the drawings, the same or similar types of elements or respectively corresponding parts are provided with the same reference numbers in order to prevent the item from needing to be reintroduced.

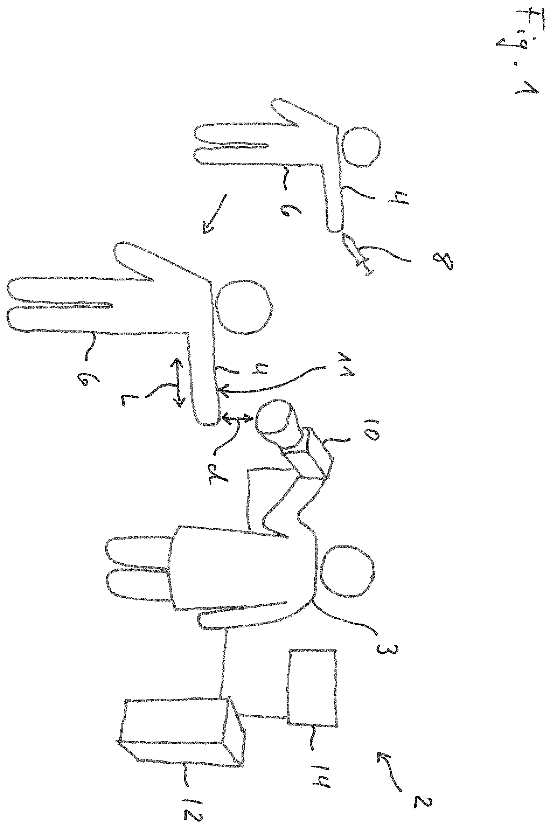

Fig. 1 illustrates an image capturing and processing device 2, which is configured to measure a fluorescence signal in a tissue of a body part 4 of a patient 6. By way of an example only, the body part 4 of the patient 6, which is inspected, is the arm. The measurement of the fluorescence signal can also be performed on other body parts 4 of the patient 6, for example the leg, a part of the head, neck, back or any other part of the body. The measurement can also be performed during open surgery. In this application scenario, the body part 4 can be for example an inner organ of the patient 6. The measurement of the fluorescent signal can also be performed during minimally invasive surgery. For this application scenario, the image capturing and processing devices 2 is at least partly integrated for example in an endoscope or laparoscope. For example, the endoscope or laparoscope comprises the image capturing device 10.

Before the measurement initially starts, a fluorescent agent 8 is administered, i.e. injected, in the tissue of the patient's body part 4. The method for measuring a fluorescence signal in the tissue of the body part 4, which will also be explained when making reference to the figures illustrating the image capturing and processing device 2, excludes the step of administering the fluorescent agent 8.

The fluorescent agent 8 is for example ICG. ICG (Indocyanine Green) is a green colored medical dye that is used for over 40 years. ICG emits fluorescent light when exited with near infrared light having a wavelength between 600 nm and 800 nm. The emitted fluorescence light is between 750 nm and 950 nm. It is also possible that the fluorescent agent 8 comprises two different medical dyes. For example, the fluorescent agent 8 can be a mixture of methylene blue and ICG.

Subsequent to the administration of the fluorescent agent 8, as it is indicated by an arrow in Fig. 1, the patient's body part 4 is inspected using an image capturing device 10, which forms part of the image capturing and processing device 2.

The image capturing device 10 is configured to image a surface 11 of the body part 4 and to detect the fluorescence signal, which results from illumination of the fluorescent agent 8 with excitation light. When the image capturing device 10 is applied in surgery, the surface 11 of the body part 4 is a surface of for example an inner organ. In this case, the surface 11 of the body part 4 is identical to the surface of the tissue, to which the fluorescent agent 8 has been administered. For emission of light having a suitable excitation wavelength, the image capturing device 10 comprises an illumination unit 16 (not shown in Fig. 1).

The captured images are communicated to a processing device 12, which also forms part of the image capturing and processing device 2. The results of the analysis are output, for example displayed on a display 14 of the processing device 12. The image capturing device 10 can be handled by a physician 3.

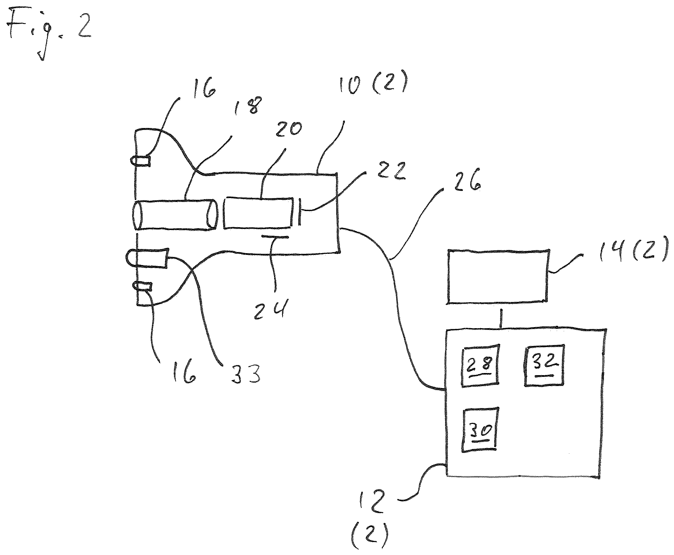

Fig. 2 is a schematic illustration showing the image capturing device 10 and the processing unit 12 of the image capturing and processing device 2 in more detail. The image capturing device 10 comprises an illumination unit 16 which is configured to illuminate the tissue with excitation light having a wavelength suitable to generate fluorescent light by exciting emission of the fluorescent agent 8. For example, a plurality of LEDs is provided in the illumination unit 16.

The image capturing device 10 further comprises an objective lens 18 through which visible light and a fluorescence light are captured. Light is guided through the objective lens 18 to a prism assembly 20. The prism assembly 20 is configured to separate fluorescent light, which is in particular in a wavelength range between 750 nm and 950 nm, from visible light that results in the visible light image. The fluorescent light is directed on a fluorescence imaging unit 22, which is for example a CCD or CMOS sensor plus additional wavelength filters and electronics, if necessary. The fluorescence imaging unit 22 is configured to capture a fluorescence image by spatially resolved measurement of the emitted light, i.e. the excited emission of the fluorescent agent 8, so as to provide the fluorescence image. Furthermore, there is a visible light imaging unit 24, which can be another CCD or CMOS sensor plus an additional different wavelength filter and electronics, if necessary. The prism assembly 20 is configured to direct visible light on the visible light imaging unit 24 so as to allow the unit to capture the visible light image of a section of a surface 11 of the patient's body part 4. Similarly, the prism assembly 20 is configured to direct fluorescent light on the fluorescence imaging unit 22. The prism assembly 20, the fluorescence imaging unit 22 and the visible light imaging unit 24 will be explained in detail further below.

In an embodiment, the image capturing device 10 is as scanning unit, for example an image line scanning unit or a LIDAR scanning unit. The image capturing device 10 can also be 3D camera, which is suitable to capture a pair of stereoscopic images from which a 3D image including depth information can be calculated. Naturally, the image capturing device 10 can be a combination of these devices.

The image data is communicated from the image capturing device 10 to the processing device 12 via a suitable data link 26, which can be a wireless datalink or a wired data link, for example a data cable.

The image capturing device 10 is configured in that the fluorescence imaging unit 22 and the visible light imaging unit 24 are operated to simultaneously capture the visible light image and the fluorescence image. In particular, the image capturing device 10 does not perform time switching between the signal of the fluorescence image and the signal of the visible light image. In other words, the sensors of the fluorescence imaging unit 22 and the visible light imaging unit 24 are exclusively used for capturing images in the respective wavelength range, which means that the sensors of the imaging units 22, 24 are used for either capturing a fluorescence image in the IR spectrum or for capturing a visible light image in the visible spectrum. The sensors 22, 24 are not used for capturing images in both wavelength ranges. This results in significant advantages. For example, the sensors can be exactly positioned in focus, which is not possible when an image sensor is used for both purposes, i.e. to capture visible light and infrared light, because the focus point for these different wavelengths typically differ in position. Furthermore, the sensor parameters can be adjusted individually, for example with respect to a required exposure time or sensor gain. Individual settings are advantageous because IR signals are typically lower than visible light signals.

The fluorescence imaging unit 22 and the visible light imaging unit 24 have a fixed spatial relationship to each other. This is because the units are arranged in one single mounting structure or frame of the image capturing device 10. Furthermore, the fluorescence imaging unit 22 and the visible light imaging unit 24 use the same objective lens 18 and prism assembly 20 for imaging of the fluorescence image and the visible light image, respectively. Due to these measures, the fluorescence imaging unit 22 and the visible light imaging 24 are configured in that a viewing direction and a perspective of the fluorescence image and the visible light image are linked via a known and constant relationship. In the given embodiment, the viewing direction of the two images are identical because both units 22, 24 image via the same objective lens 18.

The image capturing device 10 is further configured to operate the fluorescence imaging unit 22 and the visible light imaging unit 24 to repeat the capturing of the fluorescence image and the visible light image so as to provide a series of fluorescence images and a series of visible light images. This operation can be performed by the processing device 12 operating the image sensor of the fluorescence imaging unit 22 and the image sensor of visible light imaging unit 24. The series of images is typically captured while an operator or physician 3 (see Fig. 1) moves the image capturing device 10 along a longitudinal direction L of the body part 4 of the patient 6. This movement can be performed in that subsequent images of the series of images comprise overlapping parts. In other words, details which are shown in a first image of the series of images are also shown in a subsequent second image of the series. This is important for the subsequent stitching process. To safeguard that corresponding features can be found in subsequent images, the frequency of image acquisition can be set to a sufficiently high value. The capturing of the images can be manually initiated by for example the physician 3 or the capturing of images can be controlled by the image capturing device 10 in that the described prerequisite is fulfilled.

The image capturing device 10 can be further configured to acquire a position and orientation of the image capturing device 10 during this movement. For example, a position and orientation of the image capturing device 10 in a reference system of the examination room or in a reference system of the patient 6 can be determined for each image or image pair that is captured. This information can be stored and communicated to together with the image or image pair comprising the visible image and the fluorescence image. This information can be useful for the subsequent reconstruction of images so as to generate a 3D image from the series of 2D images.

Once the two series of images (i.e. a first series of visible light images and a second series of fluorescence images) or the series of image pairs (each image pair comprising a fluorescence image and a visible light image) are captured by the capturing device 10 and received in the processing device 12, the series of visible light images is processed by a stitching unit 28 (see Fig. 2). The stitching unit 28 is configured to apply a stitching algorithm on the series of visible light images to generate a large visible light image of the body part 4. The large image is "larger" in that it shows a greater section of the body part 4 of the patient 6, which is analyzed with the image capturing device 10, then a single image.

Within the context of this specification, the term "stitching" shall not be understood in that the process of stitching is limited to a combination of two or more 2D images. Stitching can also be performed on the basis of 3D images, wherein the result of this process is a larger 3D image. The process of stitching can also be performed on the basis of 2D images plus an additional information on the direction of view, from which the 2D images have been captured. Further information on the position of the image capturing device 10 can also be taken into account. As mentioned before, on the basis of these data sets, a larger 3D image can be generated, i.e. stitched together from a series of 2D images plus information on the position and orientation of the image capturing device 10. It is also possible to combine 3D scanning data, for example from a LIDAR sensor, with 2D image information. Also in this case, the result of the stitching process is a larger 3D image.

In all applications, the stitching algorithm starts with stitching of the visible light images. The stitching algorithm generates and applies a set of stitching parameters when preforming the stitching operation. The detailed operation of the stitching unit 28 will be described further below. The stitching unit 28 is configured to apply the stitching algorithm not only on the series of visible light images but also on the series of fluorescence images so as to generate a large fluorescence image. Also in this case, the process of stitching is not limited to the combination of two or more 2D images. It is also possible to generate a 3D fluorescence image in a similar way as it is described above for the visible light images.

The stitching algorithm, which is applied for stitching of the fluorescence images is the same algorithm which is used for stitching of the visible light images. Furthermore, the stitching of the fluorescence images is performed using the same set of stitching parameters which was determined when performing the stitching of the visible light images. This is possible, because there is a fixed relationship between the viewing direction and perspective of the visible light images and the fluorescence images. Naturally, if the viewing direction and perspective of the visible light images and the fluorescence images are not identical, a fixed offset or a shift in the stitching parameters has to be applied. This takes into account the known and fixed spatial relationship between the **IR** and Vis image sensors and the corresponding optics.

Subsequent to the stitching, the large visible light image and the large fluorescence image are output. For example, the images are displayed side-by-side on the display 14. Unlike traditional inspection systems, the display 14 shows a visible light image and a fluorescence image that correspond to each other. In other words, details that can be seen on the fluorescence image, for example a high fluorescence intensity that indicates an accumulation of lymphatic fluid, can be found in the patient's body part 4 exactly on the corresponding position, which is shown in the visible light image. This enables the physician 3 to exactly spot areas in which an accumulation of lymphatic fluid is present. This is very valuable information for example for a tailored and specific therapy of the patient 6.

It is also possible that the visible light image and the fluorescence image, in particular the large visible light image and the large fluorescence image are superimposed so as to provide an overlay image, in particular in a large overlay image, of the body part 4. This is performed by a superimposing unit 30 of the processing device 12. The overlay image can also be output via the display 14.

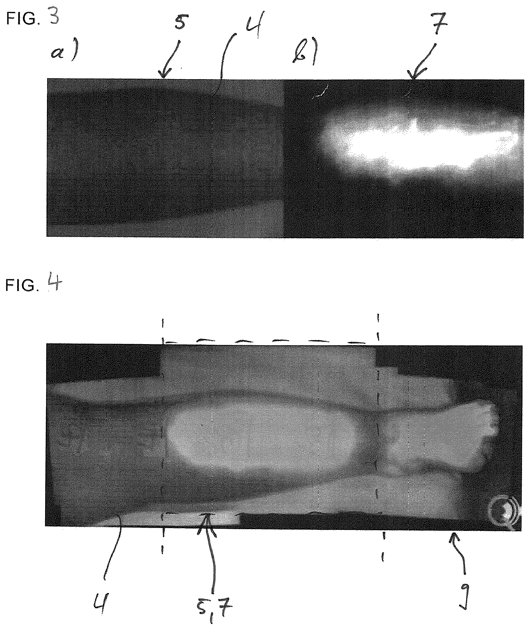

Fig. 3a) shows an example of a visible light image 5, in which a section of a surface 11 of the body part 4 of the patient 6 is visible. By way of an example only, a section of the patient's leg is depicted. Fig. 3b) shows the corresponding fluorescence image 7 determined by measuring the fluorescence signal of the fluorescence agent 8, which has been applied to the patient's tissue in the leg. A high-intensity spot or area of the fluorescence signal is visible. This strongly indicates an accumulation of lymph, which is due to a slow lymphatic transport and a possible lymphedema in the patient's leg. Advantageously, the physician 3 can now locate the area, in which the slow lymphatic transport takes place by comparing the fluorescence image 7 with the visible light image 5.

In Fig. 4, there is the overlay image 9, wherein in addition to the images shown in Figs. 3a) and 3b), stitching of the visible light images 5 and fluorescence images 7 has been performed.

An exemplary single visible light image 5 and fluorescence image 7 can also be seen in Fig. 4, it respectively projects between the dashed lines shown in the large overlay image 9. By stitching together the visible light images 5 and the fluorescence images 7, the large overlay image 9 showing almost the entire body part 4 of the patient 6 can be provided. The fluorescence signal can be shown in false color so as to clearly distinguish from features of the visible light image 5.

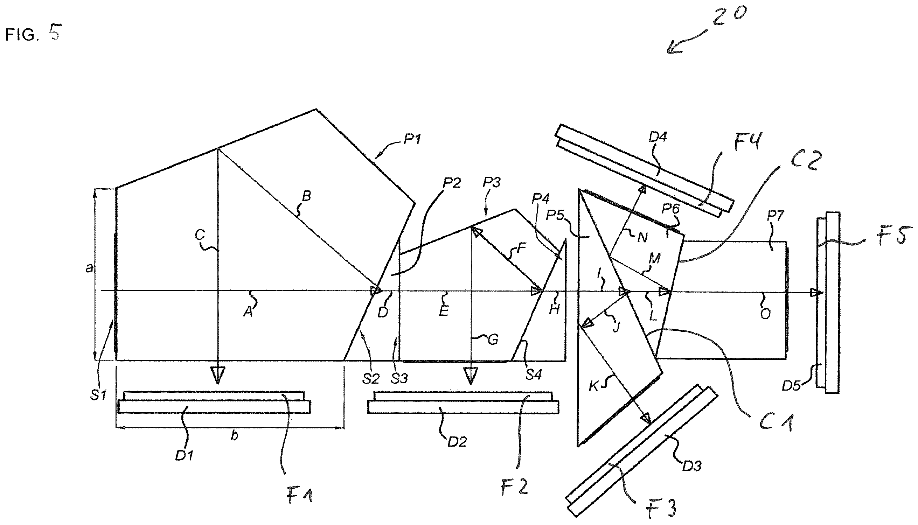

In Fig. 5, there is an embodiment of the prism assembly 20 of the image capturing device 10. A first prism P1 is a pentagonal prism. The incoming light beam A, which is visible light and fluorescence light, enters the first prism P1 via the entrance face S1 and is partially reflected on face S2, being one of the two faces not adjoining the entrance face S1. The reflected beam B is then reflected against a first one of the faces adjoining the entrance face S1. The angle of reflection can be below the critical angle, so that the reflection is not internal (the adjoining face can be coated to avoid leaking of light and reflect the required wavelength of interest). The reflected beam C then crosses the incoming light beam A and exits the first prism P1 through the second one of the faces adjoining the entrance face S1, towards sensor D1. A part of the beam A goes through face S2 and enters compensating prism P2. Two non-internal reflections can be used to direct the incoming beam A via beams B and C towards the sensor D1. Furthermore, there can be no air gaps between prisms P1 and P2 and no air gaps between prisms P3 and P4 and no air gaps between prisms P2 and P3. Prism P2 is a compensator prism which is for adjusting the individual length of the light paths from the entrance face S1 to the sensors D1..D5.

From P2, the beam D enters a second pentagonal prism P3. As in prism P1, inward reflection is used to make the beam cross itself. For brevity, the description of the beam will not be repeated, except to state that in prism P3, the beam parts E, F and G correspond to beam parts A, B and C in prism P1, respectively. Prism P3 can also not use internal reflection to reflect the incoming beam towards sensor D2. Two non-internal reflections can be used to direct the incoming beam E via beams F and G towards sensor D2.

After prism P3, there is another compensating prism P4. Finally, beam H enters the dichroic prism assembly comprising prisms P5, P6, and P7, with sensors D3, D4 and D5 respectively. The dichroic prism assembly is for splitting visible light in red, green and blue components towards respective sensors D3, D4 and D5. The light enters the prism assembly through beam I. Between P5 and P6, an optical coating C1 is placed and between prisms P6 and P7 another optical coating C2 is placed. Each optical coating C1 and C2 has a different reflectance and wavelength sensitivity. At C1, the incoming beam I is partially reflected back to the same face of the prism as through which the light entered (beam J). At that same face, the beam, now labelled K, is once again reflected towards sensor D3. The reflection from J to K is an internal reflection. Thus, sensor D3 receives light reflected by coating C1, and in analogue fashion sensor D4 receives light from beam L reflected by coating S2 (beams M and N), and sensor D5 receives light from beam O that has traversed the prism unhindered.

Between prism P4 and prism P5 there is an air gap. In the prism assembly 20, the following total path lengths can be defined for each endpoint channel (defined in terms of the sensor at the end of the channel):
Sensor D1 (e.g. first near infrared) path: A + B + C
Sensor D2 (e.g. second near infrared) path: A + D + E + F + G
Sensor D3 (e.g. red) path: A + D + E + H + I + J + K
Sensor D4 (e.g. blue) path: A + D + E + H + I + 0
Sensor D5 (e.g. green) path: A + D + E + H + I + M + N

The path lengths are matched, so that A + B + C = A+ D + E + F + G = A + D + E + H + I + J + K = A + D + E + H + I + O = A + D + E + H + I + M + N.

The matching of path lengths can comprise an adjustment for focal plane focus position differences in wavelengths to be detected at the sensors D1 - D5. That is, for example the path length towards the sensor for blue (B) light may not be exactly the same as the path length towards the sensor for red (R) light, since the ideal distances for creating a sharp, focused image are somewhat dependent on the wavelength of the light. The prisms can be configured to allow for these dependencies. D + H lengths can be adjusted and act as focus compensators due to wavelength shifts, by lateral displacement of the compensator prisms P2, P4.

A larger air gap in path I can be used for additional filters or filled with a glass compensator for focus shifts and compensation. An air gap needs to exist in that particular bottom surface of the prism P5 because of the internal reflection in the path from beam J to beam K. A space can be reserved between the prism output faces and each of the sensors D1-D5 to provide an additional filter, or should be filled up with glass compensators accordingly.

The sensors D1 and D2 are IR sensors, configured for capturing the fluorescence image 7. By way of an example, the sensors D1 and D2 plus suitable electronics are a part of the fluorescence imaging unit 22. The sensors D3, D4 and D5 are for capturing the three components of the visible light image 5. By way of an example, the sensors D3, D4 and D5 plus suitable electronics are a part of the visible light imaging unit 24. It is also possible to consider the corresponding prisms that direct the light beams on the sensors, a part of the respective unit, i.e. the fluorescence imaging unit 22 and the visible light imaging unit 24, respectively.

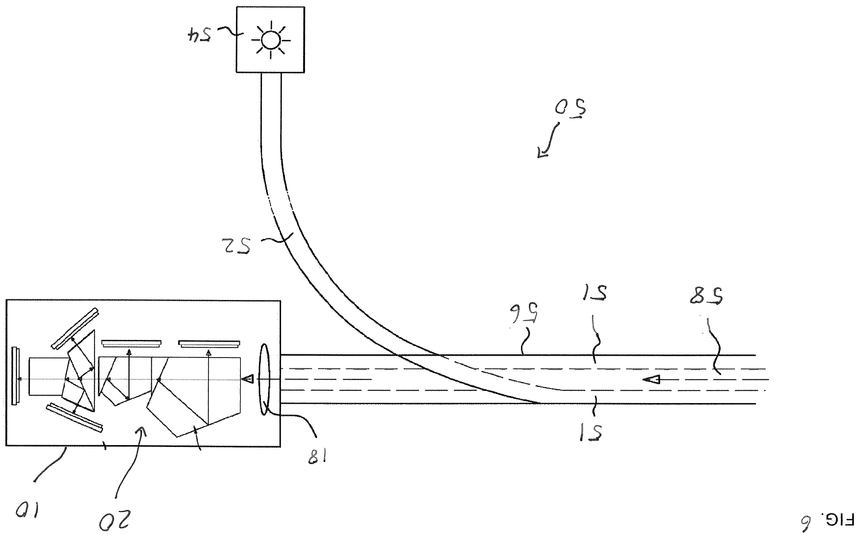

Fig. 6 schematically shows an endoscope 50 or laparoscope, according to an embodiment of the disclosure**.** The differences between lap-aroscopes and endoscopes are relatively small, when considering the aspects of the invention. Hence, where the description mentions an endoscope, a laparoscope configuration is usually also possible. By way of an example only, in the following, reference will be made to an endoscope 50.

The endoscope 50 comprises an image capturing device 10 that has been explained in further detail above. The image capturing device 10 comprises an objective lens 18 through which the fluorescent light image 7 and the visible light image 5 are captured. The objective lens 18 focuses the incoming light through the entrance face S1 of the prism assembly 20 on the sensors D1 to D5. The objective lens 18 can also be integrated in the last part of the endoscope part to match the prism back focal length.

The endoscope 50 comprises an optical fiber 52 connected to a light source 54 that couples light into the endoscope 50. The light source 54 can provide white light for illumination of the surface 11 of the body part 4 and for capturing of the visible light image 5. Furthermore, the light source 54 can be configured to emit excitation light which is suitable to excite the fluorescent dye that is applied as the fluorescent agent to emit fluorescence light. In other words, the light source 54 can be configured to emit both, visible light and light in the IR spectrum.

Inside a shaft 56 of the endoscope 50, the optical fiber 52 splits off into several fibers 51. The endoscope 50 can have a flexible shaft 56 or a rigid shaft 56. In a rigid shaft 56, a lens system consisting of lens elements and/or relay rod lenses can be used to guide the light through the shaft 56. If the endoscope 50 has a flexible shaft 56 the fiber bundle 51 can be used for guiding the light of the light source 54 to the tip of the endoscope shaft 56. For guiding light from the distal tip of the endoscope shaft 56 (is not shown in Fig. 6) coming from a field of examination to the image capturing device 10 at the proximal end of the shaft 56, a fiber bundle 58 is arranged in the shaft 56 of the endoscope 50. In another embodiment, which is not shown in the figure, the entire image capturing device 10 can be miniaturized and arranged at a distal tip or end of the endoscope shaft 56.

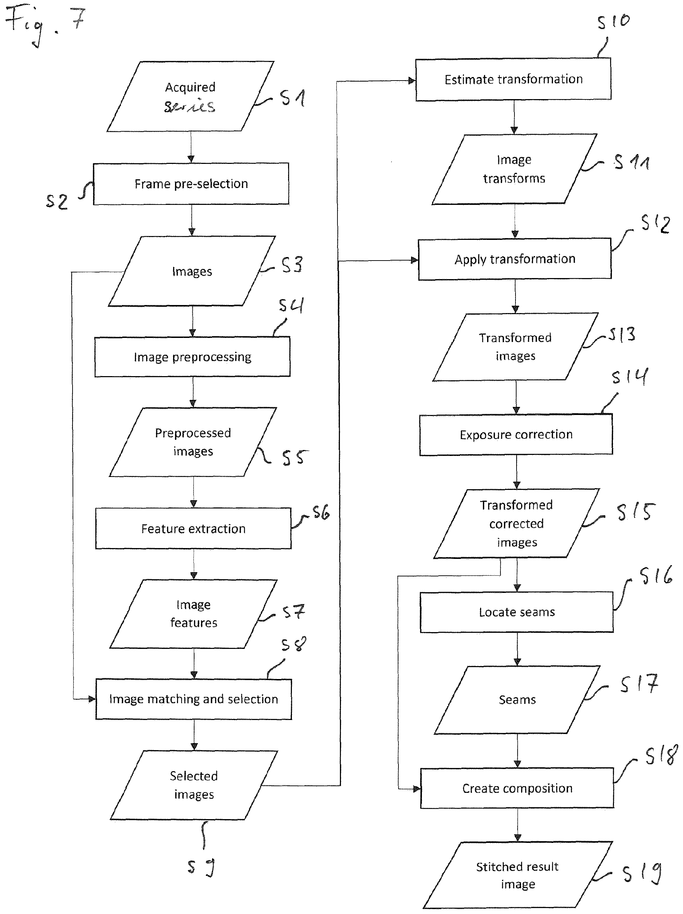

Fig. 7 shows a flowchart of the stitching algorithm, which can be used for stitching of the visible light images and the fluorescence images. The flow chart is more or less self-explanatory and will be very briefly described. Firstly, the acquired series of images (S1) is forwarded to the stitching unit 24 of the processing device 12. The algorithm then performs a frame preselection (step S2). In this preselection step, frames suitable for stitching are selected. S3 represents the selected images to be stitched, they then undergo preprocessing (step S4). In the preprocessed images (S5) a feature extraction is performed (step S6). When the image features have been extracted (S7), image matching (step S8) is performed using the images known from S3 and the extracted features from step S7. Based on the selected images (S9) a transformation of the images is estimated (step S10). This estimate of image transformation (S11), also referred to as stitching parameters, is applied (step S12). The application of the transformation results in transformed images (S13). A further image correction can be performed, for example an exposure correction (step S14). The transformed and corrected images (S15) are stitched together by locating seams (step S16), i.e. lines along which the images are joined together. The data indicating the location of the seams (S17) is used together with the transformed and corrected images (S12) to create a composition of images (step S18). In the given embodiment, this results in the large visible light image or the large fluorescence image, as the stitching results (S19).

The image capturing device 10, which is applied for capturing the visible light images 5 and the fluorescence images 7 can further comprise a measurement unit 32 which is configured to measure a distance d (see Fig. 1) between the surface 11 of the patient's body part 4, which is captured in the visible light image 5, and the image capturing device 10. A distance sensor 33, which communicates with the measurement unit 32 and which can form part of the measurement unit 32, is for example an ultrasonic sensor, a laser distance sensor or any other suitable distance measurement device. Furthermore, the image capturing device 10 is configured to output a signal, which is indicative of the measured distance d. Thereby, the measurement performed by the distance sensor 33 is communicated to a user. For example, the image capturing device 10 outputs an optical or acoustical signal giving the operator of the device 10 information on a best distance d for performance of the measurement. Performing the measurement with constant distance d significantly enhances the measurement results, because there is inter alia a homogeneous illumination.

In addition to the distance sensor 33, the image capturing device 10 can include an internal measurement unit (IMU), which can be used to collect data about a rotation in pitch, yaw, and roll as well as acceleration data in the three spatial axes (x, y and z). This information of the IMU may be used as additional data to enhance the performance of the stitching algorithm or to provide feedback to the operator to position and rotate the camera, providing better images for the stitching algorithm.

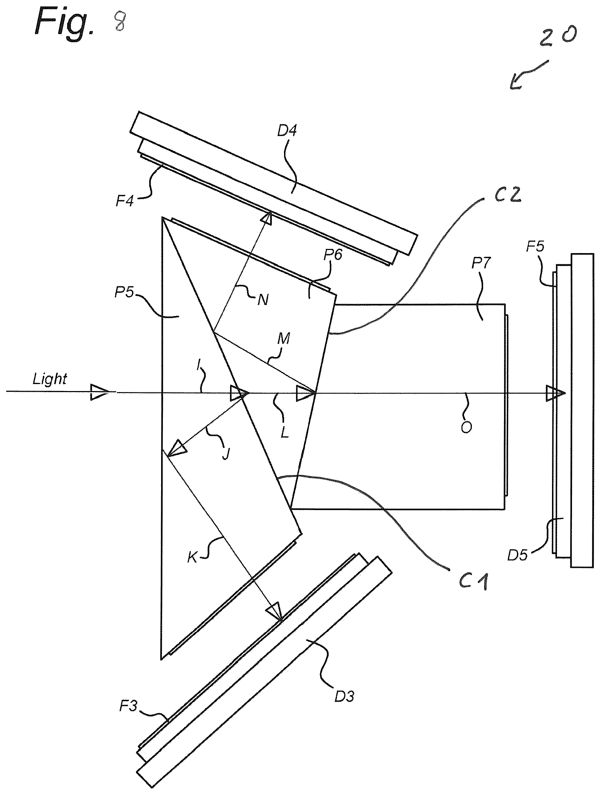

In Fig. 8, there is an embodiment of another prism assembly 20 of the image capturing device 10. The prism assembly 20 comprising prisms P5, P6, and P7, which, for example, are configured for splitting light in red, green and blue components towards respective sensors D3, D4, and D5. According to a further embodiment, the prism assembly 20 is configured to split incoming light in a green component, a red/blue component and an infrared component and to direct these towards the respective sensors D3, D4, and D5. According to still another embodiment, the prism assembly 20 is configured to split incoming light in a visible light component, which is directed to a red/green/blue sensor (RGB sensor), a first infrared component of a first wavelength or wavelength interval and a second infrared component of a second wavelength or wavelength interval, and to direct these towards the respective sensors D3, D4, and D5.

The light enters the prism assembly 20 through the arrow indicated. Between P5 and P6, an optical coating C1 is placed and between prisms P6 and P7 an optical coating C2 is placed, each optical coating C1 and C2 having a different reflectance and wavelength sensitivity. At C1, the incoming beam I is partially reflected back to the same face of the prism P5 as through which the light entered (beam J). At that same face, the beam, now labelled K, is once again reflected towards filter F3 and sensor D3. The reflection from J to K is an internal reflection. Thus, filter F3 and sensor D3 receive light reflected by coating C1, and in analogue fashion filter F4 and sensor D4 receive light from beam L reflected by coating S2 (beams M and N). Filter F5 and sensor D5 receives light from beam O that has traversed the prisms unhindered.

When making reference to the embodiment in which the incoming light is split up in a red, green and blue component, the coatings and filters are selected accordingly.

In the embodiment, in which the incoming light is separated in a green component, a red/blue component and an infrared component, the filter F3 can be a patterned filter (red/blue). In particular, there is an array of red and blue filters in an alternating pattern. The pattern can consist of groups of 2x2 pixels, which are filtered for one particular color. Filter F4 can be a green filter, which means the filter comprising only green filters. There is a single pixel grid with the light received at each pixel being filtered with a green filter. Filter F5 can be an IR filter. Each pixel is filtered with an IR filter.

In general, the coatings C1, C2 should match the filters F3, F4, F5. For example, the first coating C1 may transmit visible light while reflecting IR light, so that IR light is guided towards IR filter F3. The second coating C2 may be transparent for green light while reflecting red and blue light, so that filter F4 should be the red/blue patterned filter and F5 should be the green filter 23.

According to the further embodiment, in which in incoming light is split up in the visible light component (RGB), the first infrared component and the second infrared component, the coatings C1, C2 and the filters F3, F4, F5 are configured in that for example the sensor D4 is a color sensor (RGB sensor) for detecting the visible light image in all three colors. Furthermore, the sensor D3 can be configured for detecting fluorescence light of the first wavelength and the sensor D5 is configured for detecting fluorescence light of the second wavelength.

Similarly, when making reference to the prism assembly 20 in Fig. 5, the coatings S1, S2, S3, S4, C1 and C2 as well as the filters F1, F2, F3, F4 and F5, which are arranged in front of a respective one of the sensors D1, D2, D3, D4 and D5, can be configured in that up to four fluorescence light wavelengths can be detected. For example, the sensor D4 is a color sensor for detecting the visible light image in all three colors. The sensor D3 is for detecting fluorescence light of a first wavelength or wavelength interval, the sensor D5 is for detecting fluorescence light of a second wavelength or wavelength interval, the sensor D1 is for detecting fluorescence light of a third wavelength or wavelength interval, and the sensor D2 is for detecting fluorescence light of a fourth wavelength or wavelength interval.

Further embodiments:
Embodiment 19: A method of diagnosing lymphedema, comprising the steps of:
   - administering a fluorescent agent (8) to a body part (4),
   - measuring a fluorescence signal in a tissue of the body part (4), to which the fluorescent agent (8) has been administered, and imaging a surface (11) of the body part (4), wherein the tissue to which the fluorescent agent (8) has been added forms part of the body part (4),
   - capturing a fluorescence image by illuminating the tissue with excitation light having a wavelength suitable to generate emitted light by excited emission of the fluorescent agent (8), and by spatially resolved measurement of the emitted light so as to provide the fluorescence image,
   - capturing a visible light image of at least a section of a surface (11) of the body part (4), wherein
      a viewing direction and/or a perspective of the fluorescence image and the visible light image are linked via a known relationship and
   - repeating the steps of capturing of the fluorescence image and the visible light image to provide a series of fluorescence images and a series of visible light images,
   - applying a stitching algorithm on the series of visible light images to generate a large visible light image of the body part (4), wherein the stitching algorithm determines and applies a set of stitching parameters,
   - applying the stitching algorithm on the series of fluorescence images to generate a large fluorescence image, wherein the stitching algorithm applies the set of stitching parameters determined when performing the stitching of the visible light images
   - outputting the large visible light image and the large fluorescence image,
   - deriving a diagnostic result relative to a severity of lymphedema by analyzing the output images.
Embodiment 20: The method according to embodiment 19, wherein the fluorescent agent (8) is administered to an arm or leg of a patient by injecting the fluorescent agent in tissue between phalanges of the foot or hand of the patient.
Embodiment 21: A method of long-term therapy of lymphedema, comprising the steps of:
   - diagnosing a severity of lymphedema by performing the steps of embodiment 19 or 20 on a patient,
   - performing a therapy on the patient, the therapy being adjusted to the diagnostic result relative to the severity of lymphedema,
   - repeating the steps of diagnosing the severity of lymphedema and performing a therapy on the patient, wherein in each iteration, the therapy is adjusted to the detected severity of lymphedema.

All named characteristics, including those taken from the drawings alone, and individual characteristics, which are disclosed in combination with other characteristics, are considered alone and in combination as important to the disclosure. Embodiments according to the disclosure can be fulfilled through individual characteristics or a combination of several characteristics. Features which are combined with the wording "in particular" or "especially" are to be treated as preferred embodiments.

### List of References

- 2: image capturing and processing device
- 3: physician
- 4: body part
- 5: visible light image
- 6: patient
- 7: fluorescence image
- 8: fluorescent agent
- 9: overlay image
- 10: image capturing device
- 11: surface
- 12: processing device
- 14: display
- 16: illumination unit
- 18: objective lens
- 20: prism assembly
- 22: fluorescence imaging unit
- 24: visible light imaging unit
- 26: data link
- 28: stitching unit
- 30: superimposing unit
- 32: measurement unit
- 33: distance sensor
- 50: endoscope
- 52: optical fiber
- 51: fibers
- 54: light source
- 56: shaft
- 58: fiber bundle

- P1: first pentagonal prism
- P2, P4: compensating prism
- P3: second pentagonal prism
- P5, P6, P7: dichroic prism assembly
- A: incoming light beam
- B..O: light beams
- S1: entrance face
- D1..D5: sensors
- C1, C2: coating
- F1..F5: filter

- L: longitudinal direction
- d: distance

## Claims

1. A method of measuring a fluorescence signal in a tissue of a body part (4), to which a fluorescent agent (8) has been added, and of imaging a surface (11) of the body part (4), wherein the tissue to which the fluorescent agent (8) has been added forms part of the body part (4), the method comprising the steps of:
- capturing a fluorescence image (7) with an image capturing device (10) by illuminating the tissue with excitation light having a wavelength suitable to generate emitted light by excited emission of the fluorescent agent (8), and by spatially resolved measurement of the emitted light so as to provide the fluorescence image (7),
- capturing a visible light image (5) of at least a section of a surface (11) of the body part (4) with the image capturing device (10), wherein
a viewing direction and/or a perspective of the fluorescence image (7) and the visible light (5) image are linked via a known relationship and
the method further comprises the steps of:
- repeating the capturing of the fluorescence image (7) and the visible light image (5) to provide a series of fluorescence images (7) and a series of visible light images (5), wherein
the step of capturing the series of fluorescence images (7) and the series of visible light images (5) does not encompass capturing of images (5, 7) by manipulating the image capturing device within a living body comprising the body part (4),
- applying a stitching algorithm on the series of visible light images (7) to generate a large visible light image of the body part (4), wherein the stitching algorithm determines and applies a set of stitching parameters,
- applying the stitching algorithm on the series of fluorescence images (7) to generate a large fluorescence image, wherein the stitching algorithm applies the set of stitching parameters determined when performing the stitching of the visible light images (5) if the viewing direction and perspective of the visible light images and the fluorescence images are identical, and the stitching algorithm applies the set of stitching parameters having a fixed offset if the viewing direction and perspective of the visible light images and the fluorescence images are not identical, the offset reflecting the known relationship linking the viewing direction and perspective of the fluorescence image (7) and the visible light image (5),
- outputting the large visible light image and the large fluorescence image.

2. The method of claim 1, further comprising the step of:
- superimposing the large visible light image and the large fluorescence image to provide an overlay image (9) of the body part (4) and
- outputting the overlay image (9) as the output of the large visible light image and the large fluorescence image.

3. The method according to any of the preceding claims, wherein the viewing direction and the perspective of the fluorescence image and the visible light image are identical and in particular the fluorescence image and the visible light image are captured through one and the same objective lens (18).

4. The method according to any of the preceding claims, wherein capturing of the fluorescence image and capturing of the visible light image are performed simultaneously in absence of time-switching between a signal of the fluorescence image and a signal of the visible light image.

5. The method according to any of the preceding claims, wherein the steps of capturing the fluorescence image, illuminating the tissue with excitation light and simultaneously capturing the visible light image are performed by a single image capturing device.

6. The method according to claim 5, further comprising the steps of:
- measuring a distance (d) between a surface (11) of the body part (4), which is captured in the visible light image, and the capturing device, wherein in particular the method further comprising the step of outputting a signal by the image capturing device (10), which is indicative of the measured distance (d)..

7. The method of claim 6, further comprising the steps of:
- repeatedly capturing the fluorescence image and the visible light image of the same section of the surface (11) of the body part (4) while measuring the distance (d), wherein a plurality of sets of fluorescence and visible light images are captured at different distances (d),
analyzing the sets of images in view of imaging quality and determining a best matching distance (d*) resulting in the highest quality of images, wherein in particular the image capturing device (10) outputs a signal, which is indicative of a deviation of the measured distance (d) from the best matching distance (d*).

8. The method according to any of the preceding claims, wherein the measurement of the fluorescence signal is performed on a tissue, to which at least a first and a second fluorescent agent (8) has been added, wherein the step of capturing the fluorescence image comprises:
- capturing a first fluorescence image in a first wavelength range, which is generated by illuminating the tissue with first excitation light having a first wavelength suitable to generate emitted light by a first excited emission of the first fluorescent agent (8), and
- capturing a second fluorescence image in a second wavelength range, which is generated by illuminating the tissue with second excitation light having a second wavelength suitable to generate emitted light by a second excited emission of the second fluorescent agent (8), and the method further comprises the steps of
- repeating the capturing of the first and the second fluorescence image to provide a first and a second series of fluorescence images,
- applying the stitching algorithm on the first and second series of fluorescence images to generate a first and a second large fluorescence image, wherein the stitching algorithm applies the set of stitching parameters determined when performing the stitching of the visible light images (5),
- outputting the first and the second large fluorescence image.

9. An image capturing and processing device (2) configured to measure a fluorescence signal in a tissue of a body part (4), to which a fluorescent agent (8) has been added, and configured to image a surface (11) of the body part (4), wherein the tissue to which the fluorescent agent (8) has been added forms part of the body part (4), the image capturing and processing device (2) comprising an image capturing device (10), which further comprises
- an illumination unit (16) configured to illuminate the tissue with excitation light having a wavelength suitable to generate emitted light by excited emission of the fluorescent agent (8),
- a fluorescence imaging unit (22) configured to capture a fluorescence image by spatially resolved measurement of the emitted light so as to provide a fluorescence image,
- a visible light imaging unit (24) configured to capture a visible light image of a section of a surface (11) of the body part (4),
wherein
the fluorescence imaging unit (22) and the visible light imaging unit (24) are configured in that a viewing direction and/or a perspective of the fluorescence image and the visible light image are linked via a known relationship, wherein
- the fluorescence imaging unit (22) and the visible light imaging unit (24) are further configured to repeat capturing of the fluorescence image and the visible light image to provide a series of fluorescence images and a series of visible light images, the image capturing and processing device (2) comprising a processing unit (12), which further comprises
- a stitching unit (28) configured to apply a stitching algorithm on the series of visible light images to generate a large visible light image of the body part (4), the stitching algorithm determining and applying a set of stitching parameters,
wherein the stitching unit (28) is further configured to apply the stitching algorithm on the series of fluorescence images to generate a large fluorescence image, wherein the stitching algorithm applies the set of stitching parameters determined when performing the stitching of the visible light images if the viewing direction and perspective of the visible light images and the fluorescence images are identical, and the stitching unit (28) is configured to apply the stitching algorithm in that it applies the set of stitching parameters having a fixed offset if the viewing direction and perspective of the visible light images and the fluorescence images are not identical, the offset reflecting the known relationship linking the viewing direction and perspective of the fluorescence image (7) and the visible light image (5),
- an output unit configured to output the large visible light image and the large fluorescence image.

10. The device according to claim 9, further comprising:
- a superimposing unit configured to superimpose the large visible light image and the large fluorescence image to provide an overlay image of the body part (4), wherein the output unit is further configured to output the overlay image as output of the large visible light image and the large fluorescence image.

11. The device (2) according to claim 9 or 10, wherein the fluorescence imaging unit (22) and the visible light imaging unit (24) are configured in that the viewing direction and the perspective of the fluorescence image and the visible light image are identical, wherein in particular the fluorescence imaging unit (22) and the visible light imaging unit (24) are configured in that the fluorescence image and the visible light image are captured through one and the same objective lens (18).

12. The device (2) according to any of claims 9 to 11, wherein the fluorescence imaging unit (22) and the visible light imaging unit (24) are configured to capture the fluorescence image and the visible light image simultaneously, in absence of time-switching between a signal of the fluorescence image and a signal of the visible light image.

13. The device (2) according to any of claims 9 to 12, wherein the image capturing device (10) comprises a dichroic prism assembly configured to receive fluorescent light and visible light through an entrance face, comprising: a first prism (P1), a second prism (P3), a first compensator prism (P2) located between the first prism (P1) and the second prism (P3),
a further dichroic prism assembly (P5, P6, P7) for splitting the visible light in three light components, and a second compensator prism (P4) located between the second prism (P3) and the further dichroic prism assembly (P5, P6, P7),
wherein the first prism (P1) and the second prism (P3) each have a cross section with at least five corners, each corner having an inside angle of at least 90 degrees, wherein the corners of the first prism (P1) and the second prism (P3) each have a respective entrance face (S1, S2) and a respective exit face, and are each designed so that an incoming beam which enters the entrance face of the respective prism in a direction parallel to a normal of said entrance face is reflected twice inside the respective prism and exits the respective prism through its exit face parallel to a normal of said exit face,
wherein the normal of the entrance face and the normal of the exit face of the respective prism are perpendicular to each other; and
wherein, when light enters the first prism (P1) through the entrance face, the light is partially reflected towards the exit face of the first prism (P1) thereby traveling a first path length from the entrance face of the first prism (P1) to the exit face of the first prism (P1 ), and the light partially enters the second prism (P3) via the first compensator prism (P2) and is partially reflected towards the exit face of the second prism (P3), thereby traveling a second path length from the entrance face of the first prism (P1) to the exit face of the second prism (P3),
wherein the first prism (P1) is larger than the second prism (P3) so that the first and the second path lengths are the same.

14. The device (2) according to any of claims 9 to 13, wherein the illumination unit (16), the fluorescence imaging unit (22) and the visible light imaging unit (24) are arranged in a single image capturing device (10), which further comprises a measurement unit (32) configured to measure a distance (d) between the surface (11) of the body part (4), which is captured in the visible light image, wherein in particular the image capturing device (10) is further configured to output a signal, which is indicative of the measured distance (d),

15. An endoscope or laparoscope being configured as the image capturing device (10) in the image capturing and processing device (2) according to any of claims 9 to 14.

## Patentansprüche

1. Verfahren zur Messung eines Fluoreszenzsignals in einem Gewebe eines Körperteils (4), dem ein fluoreszierendes Mittel (8) hinzugefügt wurde, und zur Abbildung einer Oberfläche (11) des Körperteils (4), wobei das Gewebe, dem das fluoreszierende Mittel (8) hinzugefügt wurde, einen Teil des Körperteils (4) bildet, wobei das Verfahren die folgenden Schritte umfasst:
- Aufnehmen eines Fluoreszenzbilds (7) mit einer Bilderfassungseinrichtung (10) durch Beleuchten des Gewebes mit Anregungslicht, das eine Wellenlänge aufweist, die dafür geeignet ist, emittiertes Licht durch angeregte Emission des fluoreszierenden Mittels (8) zu erzeugen, und durch räumlich aufgelöste Messung des emittierten Lichts, sodass das Fluoreszenzbild (7) bereitgestellt wird,
- Aufnehmen eines Bilds (5) sichtbaren Lichts zumindest eines Abschnitts einer Oberfläche (11) des Körperteils (4) mit der Bilderfassungseinrichtung (10), wobei
eine Blickrichtung und/oder eine Perspektive des Fluoreszenzbilds (7) und des Bilds (5) sichtbaren Lichts über eine bekannte Beziehung verknüpft sind und
das Verfahren ferner die folgenden Schritte umfasst:
- Wiederholen des Aufnehmens des Fluoreszenzbilds (7) und des Bilds (5) sichtbaren Lichts, sodass eine Reihe von Fluoreszenzbildern (7) und eine Reihe von Bildern (5) sichtbaren Lichts bereitgestellt werden, wobei
der Schritt des Aufnehmens der Reihe von Fluoreszenzbildern (7) und der Reihe von Bildern (5) sichtbaren Lichts kein Aufnehmen von Bildern (5, 7) durch Betätigen der Bilderfassungseinrichtung innerhalb eines lebenden Körpers, der den Körperteil (4) umfasst, umfasst,
- Anwenden eines Stitching-Algorithmus auf die Reihe von Bildern (7) sichtbaren Lichts, sodass ein großes Bild sichtbaren Lichts des Körperteils (4) erzeugt wird, wobei der Stitching-Algorithmus einen Satz von Stitching-Parametern bestimmt und anwendet,
- Anwenden des Stitching-Algorithmus auf die Reihe von Fluoreszenzbildern (7), sodass ein großes Fluoreszenzbild erzeugt wird, wobei der Stitching-Algorithmus den bestimmten Satz von Stitching-Parametern beim Ausführen des Stitchings der Bilder (5) sichtbaren Lichts anwendet, wenn die Blickrichtung und die Perspektive der Bilder sichtbaren Lichts und der Fluoreszenzbilder identisch sind, und der Stitching-Algorithmus den Satz von Stitching-Parametern mit einem festen Versatz anwendet, wenn die Blickrichtung und die Perspektive der Bilder sichtbaren Lichts und der Fluoreszenzbilder nicht identisch sind, wobei der Versatz die bekannte Beziehung widerspiegelt, die die Blickrichtung und die Perspektive des Fluoreszenzbilds (7) und des Bilds (5) sichtbaren Lichts verknüpft,
- Ausgeben des großen Bilds sichtbaren Lichts und des großen Fluoreszenzbilds.

2. Verfahren nach Anspruch 1, das ferner den folgenden Schritt umfasst:
- Überlagern des großen Bilds sichtbaren Lichts und des großen Fluoreszenzbilds, sodass ein Überlagerungsbild (9) des Körperteils (4) bereitgestellt wird, und
- Ausgeben des Überlagerungsbilds (9) als die Ausgabe des großen Bilds sichtbaren Lichts und des großen Fluoreszenzbilds.

3. Verfahren nach einem beliebigen der vorhergehenden Ansprüche, wobei die Blickrichtung und die Perspektive des Fluoreszenzbilds und des Bilds sichtbaren Lichts identisch sind und das Fluoreszenzbild und das Bild sichtbaren Lichts insbesondere durch ein und dieselbe Objektivlinse (18) hindurch aufgenommen werden.

4. Verfahren nach einem beliebigen der vorhergehenden Ansprüche, wobei das Aufnehmen des Fluoreszenzbilds und das Aufnehmen des Bilds sichtbaren Lichts gleichzeitig ohne zeitliches Umschalten zwischen einem Signal des Fluoreszenzbilds und einem Signal des Bilds sichtbaren Lichts ausgeführt werden.

5. Verfahren nach einem beliebigen der vorhergehenden Ansprüche, wobei die Schritte des Aufnehmens des Fluoreszenzbilds, des Beleuchtens des Gewebes mit Anregungslicht und des gleichzeitigen Aufnehmens des Bilds sichtbaren Lichts von einer einzelnen Bilderfassungseinrichtung ausgeführt werden.

6. Verfahren nach Anspruch 5, das ferner die folgenden Schritte umfasst:
- Messen eines Abstands (d) zwischen einer Oberfläche (11) des Körperteils (4), die in dem Bild sichtbaren Lichts erfasst ist, und der Erfassungseinrichtung, wobei das Verfahren insbesondere ferner den Schritt des Ausgebens eines Signals, das den gemessenen Abstand (d) angibt, mittels der Bilderfassungseinrichtung (10) umfasst.

7. Verfahren nach Anspruch 6, das ferner die folgenden Schritte umfasst:
- wiederholtes Aufnehmen des Fluoreszenzbilds und des Bilds sichtbaren Lichts desselben Abschnitts der Oberfläche (11) des Körperteils (4) bei gleichzeitigem Messen des Abstands (d), wobei eine Vielzahl von Sätzen von Fluoreszenzbildern und Bildern sichtbaren Lichts in unterschiedlichen Abständen (d) aufgenommen werden,
Analysieren der Sätze von Bildern hinsichtlich der Bildqualität und Bestimmen eines am besten passenden Abstands (d*), der zu der höchsten Qualität von Bildern führt, wobei die Bilderfassungseinrichtung (10) insbesondere ein Signal ausgibt, das eine Abweichung des gemessenen Abstands (d) von dem am besten passenden Abstand (d*) angibt.

8. Verfahren nach einem beliebigen der vorhergehenden Ansprüche, wobei die Messung des Fluoreszenzsignals an einem Gewebe ausgeführt wird, dem mindestens ein erstes und ein zweites fluoreszierendes Mittel (8) hinzugefügt wurden, wobei der Schritt des Aufnehmens des Fluoreszenzbilds Folgendes umfasst:
- Aufnehmen eines ersten Fluoreszenzbilds in einem ersten Wellenlängenbereich, der durch Beleuchten des Gewebes mit erstem Anregungslicht erzeugt wird, das eine erste Wellenlänge aufweist, die dafür geeignet ist, emittiertes Licht durch eine erste angeregte Emission des ersten fluoreszierenden Mittels (8) zu erzeugen, und
- Aufnehmen eines zweiten Fluoreszenzbilds in einem zweiten Wellenlängenbereich, der durch Beleuchten des Gewebes mit zweitem Anregungslicht erzeugt wird, das eine zweite Wellenlänge aufweist, die dafür geeignet ist, emittiertes Licht durch eine zweite angeregte Emission des zweiten fluoreszierenden Mittels (8) zu erzeugen, und wobei das Verfahren ferner die folgenden Schritte umfasst
- Wiederholen des Aufnehmens des ersten und des zweiten Fluoreszenzbilds, sodass eine erste und eine zweite Reihe von Fluoreszenzbildern bereitgestellt werden,
- Anwenden des Stitching-Algorithmus auf die erste und die zweite Reihe von Fluoreszenzbildern, sodass ein erstes und ein zweites großes Fluoreszenzbild erzeugt werden, wobei der Stitching-Algorithmus den bestimmten Satz von Stitching-Parametern beim Ausführen des Stitchings der Bilder (5) sichtbaren Lichts anwendet,
- Ausgeben des ersten und des zweiten großen Fluoreszenzbilds.

9. Bilderfassungs- und Verarbeitungseinrichtung (2), die so ausgestaltet ist, dass sie ein Fluoreszenzsignal in einem Gewebe eines Körperteils (4), dem ein fluoreszierendes Mittel (8) hinzugefügt wurde, misst, und so ausgestaltet ist, dass sie eine Oberfläche (11) des Körperteils (4) abbildet, wobei das Gewebe, dem das fluoreszierende Mittel (8) hinzugefügt wurde, einen Teil des Körperteils (4) bildet, wobei die Bilderfassungs- und Verarbeitungseinrichtung (2) eine Bilderfassungseinrichtung (10) umfasst, die ferner Folgendes umfasst
- eine Beleuchtungseinheit (16), die so ausgestaltet ist, dass sie das Gewebe mit Anregungslicht beleuchtet, das eine Wellenlänge aufweist, die dafür geeignet ist, emittiertes Licht durch angeregte Emission des fluoreszierenden Mittels (8) zu erzeugen,
- eine Einheit (22) zur Abbildung von Fluoreszenz, die so ausgestaltet ist, dass sie ein Fluoreszenzbild durch räumlich aufgelöste Messung des emittierten Lichts aufnimmt, sodass ein Fluoreszenzbild bereitgestellt wird,
- eine Einheit (24) zur Abbildung von sichtbarem Licht, die so ausgestaltet ist, dass sie ein Bild sichtbaren Lichts eines Abschnitts einer Oberfläche (11) des Körperteils (4) aufnimmt, wobei
die Einheit (22) zur Abbildung von Fluoreszenz und die Einheit (24) zur Abbildung von sichtbarem Licht derart ausgestaltet sind, dass eine Blickrichtung und/oder eine Perspektive des Fluoreszenzbilds und des Bilds sichtbaren Lichts über eine bekannte Beziehung verknüpft sind, wobei
- die Einheit (22) zur Abbildung von Fluoreszenz und die Einheit (24) zur Abbildung von sichtbarem Licht ferner so ausgestaltet sind, dass sie das Aufnehmen des Fluoreszenzbilds und des Bilds sichtbaren Lichts wiederholen, sodass eine Reihe von Fluoreszenzbildern und eine Reihe von Bildern sichtbaren Lichts bereitgestellt werden, wobei die Bilderfassungs- und Verarbeitungseinrichtung (2) eine Verarbeitungseinheit (12) umfasst, die ferner Folgendes umfasst
- eine Stitching-Einheit (28), die so ausgestaltet ist, dass sie einen Stitching-Algorithmus auf die Reihe von Bildern sichtbaren Lichts anwendet, sodass ein großes Bild sichtbaren Lichts des Körperteils (4) erzeugt wird, wobei der Stitching-Algorithmus einen Satz von Stitching-Parametern bestimmt und anwendet,
wobei die Stitching-Einheit (28) ferner so ausgestaltet ist, dass sie den Stitching-Algorithmus auf die Reihe von Fluoreszenzbildern anwendet, sodass ein großes Fluoreszenzbild erzeugt wird, wobei der Stitching-Algorithmus den bestimmten Satz von Stitching-Parametern beim Ausführen des Stitchings der Bilder sichtbaren Lichts anwendet, wenn die Blickrichtung und die Perspektive der Bilder sichtbaren Lichts und der Fluoreszenzbilder identisch sind, und die Stitching-Einheit (28) so ausgestaltet ist, dass sie den Stitching-Algorithmus derart anwendet, dass sie den Satz von Stitching-Parametern mit einem festen Versatz anwendet, wenn die Blickrichtung und die Perspektive der Bilder sichtbaren Lichts und der Fluoreszenzbilder nicht identisch sind, wobei der Versatz die bekannte Beziehung widerspiegelt, die die Blickrichtung und die Perspektive des Fluoreszenzbilds (7) und des Bilds (5) sichtbaren Lichts verknüpft,
- eine Ausgabeeinheit, die so ausgestaltet ist, dass sie das große Bild sichtbaren Lichts und das große Fluoreszenzbild ausgibt.

10. Einrichtung nach Anspruch 9, ferner umfassend:
- eine Überlagerungseinheit, die so ausgestaltet ist, dass sie das große Bild sichtbaren Lichts und das große Fluoreszenzbild überlagert, sodass ein Überlagerungsbild des Körperteils (4) bereitgestellt wird, wobei die Ausgabeeinheit ferner so ausgestaltet ist, dass sie das Überlagerungsbild als Ausgabe des großen Bilds sichtbaren Lichts und des großen Fluoreszenzbilds ausgibt.

11. Einrichtung (2) nach Anspruch 9 oder 10, wobei die Einheit (22) zur Abbildung von Fluoreszenz und die Einheit (24) zur Abbildung von sichtbarem Licht derart ausgestaltet sind, dass die Blickrichtung und die Perspektive des Fluoreszenzbilds und des Bilds sichtbaren Lichts identisch sind, wobei die Einheit (22) zur Abbildung von Fluoreszenz und die Einheit (24) zur Abbildung von sichtbarem Licht insbesondere derart ausgestaltet sind, dass das Fluoreszenzbild und das Bild sichtbaren Lichts durch ein und dieselbe Objektivlinse (18) hindurch aufgenommen werden.

12. Einrichtung (2) nach einem beliebigen der Ansprüche 9 bis 11, wobei die Einheit (22) zur Abbildung von Fluoreszenz und die Einheit (24) zur Abbildung von sichtbarem Licht so ausgestaltet sind, dass sie das Fluoreszenzbild und das Bild sichtbaren Lichts gleichzeitig, ohne zeitliches Umschalten zwischen einem Signal des Fluoreszenzbilds und einem Signal des Bilds sichtbaren Lichts, aufnehmen.

13. Einrichtung (2) nach einem beliebigen der Ansprüche 9 bis 12, wobei die Bilderfassungseinrichtung (10) umfasst:
eine Anordnung dichroitischer Prismen, die so ausgestaltet ist, dass sie Fluoreszenzlicht und sichtbares Licht durch eine Eintrittsfläche hindurch empfängt, umfassend: ein erstes Prisma (P1), ein zweites Prisma (P3), ein erstes Ausgleichsprisma (P2), das sich zwischen dem ersten Prisma (P1) und dem zweiten Prima (P3) befindet,
eine weitere Anordnung dichroitischer Prismen (P5, P6, P7) zum Aufspalten des sichtbaren Lichts in drei Lichtkomponenten und ein zweites Ausgleichsprisma (P4), das sich zwischen dem zweiten Prisma (P3) und der weiteren Anordnung dichroitischer Prismen (P5, P6, P7) befindet, umfasst,
wobei das erste Prisma (P1) und das zweite Prisma (P3) jeweils einen Querschnitt mit mindestens fünf Ecken aufweisen, wobei jede Ecke einen Innenwinkel von mindestens 90 Grad aufweist, wobei die Ecken des ersten Prismas (P1) und des zweiten Prismas (P3) jeweils eine jeweilige Eintrittsfläche (S1, S2) und eine jeweilige Austrittsfläche aufweisen und jeweils so ausgebildet sind, dass ein eintreffender Strahl, der über die Eintrittsfläche des jeweiligen Prismas in einer Richtung parallel zu einer Normalen der Eintrittsfläche eintritt, im Inneren des jeweiligen Prismas zweimal reflektiert wird und aus dem jeweiligen Prisma durch dessen Austrittsfläche hindurch parallel zu einer Normalen der Austrittsfläche austritt,
wobei die Normale der Eintrittsfläche und die Normale der Austrittsfläche des jeweiligen Prismas senkrecht zueinander sind; und
wobei, wenn Licht durch die Eintrittsfläche hindurch in das erste Prisma (P1) eintritt, das Licht teilweise hin zu der Austrittsfläche des ersten Prismas (P1) reflektiert wird, wodurch es eine erste Weglänge von der Eintrittsfläche des ersten Prismas (P1) zu der Austrittsfläche des ersten Prismas (P1) zurücklegt, und das Licht über das erste Ausgleichsprisma (P2) teilweise in das zweite Prisma (P3) eintritt und teilweise hin zu der Austrittsfläche des zweiten Prismas (P3) reflektiert wird, wodurch es eine zweite Weglänge von der Eintrittsfläche des ersten Prismas (P1) zu der Austrittsfläche des zweiten Prismas (P3) zurücklegt,
wobei das erste Prisma (P1) größer als das zweite Prisma (P3) ist, sodass die erste und die zweite Weglänge gleich sind.

14. Einrichtung (2) nach einem beliebigen der Ansprüche 9 bis 13, wobei die Beleuchtungseinheit (16), die Einheit (22) zur Abbildung von Fluoreszenz und die Einheit (24) zur Abbildung von sichtbarem Licht in einer einzelnen Bilderfassungseinrichtung (10) angeordnet sind, die ferner eine Messeinheit (32) umfasst, die so ausgestaltet ist, dass sie einen Abstand (d) zwischen der Oberfläche (11) des Körperteils (4), die in dem Bild sichtbaren Lichts erfasst ist, misst, wobei die Bilderfassungseinrichtung (10) ferner insbesondere so ausgestaltet ist, dass sie ein Signal ausgibt, das den gemessenen Abstand (d) angibt,

15. Endoskop oder Laparoskop, das als die Bilderfassungseinrichtung (10) in der Bilderfassungs- und Verarbeitungseinrichtung (2) nach einem beliebigen der Ansprüche 9 bis 14 ausgestaltet ist.

## Revendications

1. Un procédé de mesure d'un signal de fluorescence dans un tissu d'une partie corporelle (4) auquel un agent fluorescent (8) a été ajouté, et de capture d'image d'une surface (11) de la partie corporelle (4), le tissu auquel l'agent fluorescent (8) a été ajouté faisant partie de la partie corporelle (4), le procédé comprenant les étapes suivantes :
- capturer une image en fluorescence (7) avec un dispositif (10) de capture d'image en éclairant le tissu avec une lumière d'excitation ayant une longueur d'onde appropriée pour générer une lumière émise par émission excitée de l'agent fluorescent (8), et en mesurant de manière spatialement résolue la lumière émise de façon à produire l'image en fluorescence (7),
- capturer une image en lumière visible (5) d'au moins une section d'une surface (11) de la partie corporelle (4) avec le dispositif (10) de capture d'image,
une direction d'observation et/ou une perspective de l'image en fluorescence (7) et de l'image en lumière visible (5) étant liées par une relation connue, et le procédé comprenant en outre les étapes suivantes :
- répéter la capture de l'image en fluorescence (7) et de l'image en lumière visible (5) afin de produire une série d'images en fluorescence (7) et une série d'images en lumière visible (5),
l'étape consistant à capturer la série d'images en fluorescence (7) et la série d'images en lumière visible (5) n'englobe pas la capture d'images (5, 7) en manipulant le dispositif de capture d'images à l'intérieur d'un être vivant comprenant la partie corporelle (4),
- appliquer un algorithme d'assemblage à la série d'images en lumière visible (7) afin de générer une grande image en lumière visible de la partie corporelle (4), l'algorithme d'assemblage déterminant et appliquant un ensemble de paramètres d'assemblage,
- appliquer l'algorithme d'assemblage à la série d'images en fluorescence (7) afin de générer une grande image en fluorescence, l'algorithme d'assemblage appliquant l'ensemble de paramètres d'assemblage déterminé lors de la réalisation de l'assemblage des images en lumière visible (5) si la direction d'observation et la perspective des images en lumière visible et des images en fluorescence sont identiques, et l'algorithme d'assemblage appliquant l'ensemble de paramètres d'assemblage ayant un décalage fixe si la direction d'observation et la perspective des images en lumière visible et des images en fluorescence ne sont pas identiques, le décalage reflétant la relation connue reliant la direction d'observation et la perspective de l'image en fluorescence (7) et de l'image en lumière visible (5),
- émettre la grande image en lumière visible et la grande image en fluorescence.

2. Le procédé selon la revendication 1, comprenant en outre l'étape suivante :
- superposer la grande image en lumière visible et la grande image en fluorescence pour fournir une image superposée (9) de la partie corporelle (4), et
- émettre l'image superposée (9) en tant qu'émission de la grande image en lumière visible et de la grande image en fluorescence.

3. Le procédé selon l'une quelconque des revendications précédentes, dans lequel la direction d'observation et la perspective de l'image en fluorescence et de l'image en lumière visible sont identiques et, en particulier, l'image en fluorescence et l'image en lumière visible sont capturées à travers un seul et même objectif (18).

4. Le procédé selon l'une quelconque des revendications précédentes, dans lequel la capture de l'image en fluorescence et la capture de l'image en lumière visible sont effectuées simultanément en l'absence de commutation temporelle entre un signal de l'image en fluorescence et un signal de l'image en lumière visible.

5. Le procédé selon l'une quelconque des revendications précédentes, dans lequel les étapes de capture de l'image en fluorescence, d'éclairage du tissu avec une lumière d'excitation et de capture simultanée de l'image en lumière visible sont mises en œuvre par un seul dispositif de capture d'image.

6. Le procédé selon la revendication 5, comprenant en outre les étapes suivantes :
- mesurer une distance (d) entre une surface (11) de la partie corporelle (4), qui est capturée dans l'image en lumière visible, et le dispositif de capture, le procédé comprenant en particulier l'étape consistant à émettre un signal par le dispositif (10) de capture d'image, qui est indicatif de la distance mesurée (d).

7. Le procédé selon la revendication 6, comprenant en outre les étapes suivantes :
- capturer de manière répétée l'image en fluorescence et l'image en lumière visible de la même section de la surface (11) de la partie corporelle (4) tout en mesurant la distance (d), une pluralité d'ensembles d'images en fluorescence et de lumière visible étant capturés à différentes distances (d),
- analyser les ensembles d'images en fonction de la qualité de l'image et déterminer une distance optimale (d*) permettant d'obtenir la meilleure qualité d'image, le dispositif de capture d'images (10) émettant notamment un signal qui est indicateur d'un écart entre la distance mesurée (d) et la distance optimale (d%*).

8. Le procédé selon l'une quelconque des revendications précédentes, dans lequel la mesure du signal de fluorescence est effectuée sur un tissu auquel au moins un premier et un deuxième agents fluorescents (8) ont été ajoutés, l'étape de capture de l'image en fluorescence comprenant :
- le fait de capturer une première image en fluorescence dans une première gamme de longueurs d'onde, qui est générée en éclairant le tissu avec une première lumière d'excitation ayant une première longueur d'onde appropriée pour générer une lumière émise par une première émission excitée du premier agent fluorescent (8), et
- le fait de capturer une deuxième image en fluorescence dans une deuxième gamme de longueurs d'onde, qui est générée en éclairant le tissu avec une deuxième lumière d'excitation ayant une deuxième longueur d'onde adaptée pour générer une lumière émise par une deuxième émission excitée du deuxième agent fluorescent (8), et le procédé comprend en outre les étapes suivantes :
- le fait de répéter la capture de la première et de la deuxième image en fluorescence pour fournir une première et une deuxième série d'images en fluorescence,
- le fait d'appliquer l'algorithme d'assemblage sur les première et deuxième séries d'images en fluorescence pour générer une première et une deuxième grande image en fluorescence, l'algorithme d'assemblage appliquant l'ensemble de paramètres d'assemblage déterminés lors de l'assemblage des images en lumière visible (5),
- le fait d'émettre la première et la deuxième grandes images en fluorescence.

9. Un dispositif (2) de capture et de traitement d'images, conçu pour mesurer un signal de fluorescence dans un tissu d'une partie corporelle (4) auquel un agent fluorescent (8) a été ajouté, et conçu pour capturer une image d'une surface (11) de la partie corporelle (4), le tissu auquel l'agent fluorescent (8) a été ajouté faisant partie de la partie corporelle (4), le dispositif (2) de capture et de traitement d'images comprenant un dispositif (10) de capture d'images, qui comprend en outre
- une unité d'éclairage (16), conçue pour éclairer le tissu avec une lumière d'excitation ayant une longueur d'onde adaptée pour générer une lumière émise par émission excitée de l'agent fluorescent (8),
- une unité (22) de capture d'image en fluorescence, conçue pour capturer une image en fluorescence par mesure spatialement résolue de la lumière émise afin de produire une image en fluorescence,
- une unité (24) de capture d'image en lumière visible, conçue pour capturer une image en lumière visible d'une section d'une surface (11) de la partie corporelle (4),
l'unité (22) de capture d'image en fluorescence et l'unité (24) de capture d'image en lumière visible étant conçues de telle sorte qu'une direction d'observation et/ou une perspective de l'image en fluorescence et de l'image en lumière visible soient liées par une relation connue, dans laquelle
- l'unité (22) de capture d'image en fluorescence et l'unité (24) de capture d'image en lumière visible sont en outre conçues pour répéter la capture de l'image en fluorescence et de l'image en lumière visible afin de produire une série d'images par fluorescence et une série d'images par lumière visible, le dispositif (2) de capture et de traitement d'images comprenant une unité de traitement (12), qui comprend en outre
- une unité d'assemblage (28), conçue pour appliquer un algorithme d'assemblage à la série d'images en lumière visible afin de générer une grande image en lumière visible de la partie corporelle (4), l'algorithme d'assemblage déterminant et appliquant un ensemble de paramètres d'assemblage,
l'unité d'assemblage (28) étant en outre conçue pour appliquer l'algorithme d'assemblage à la série d'images en fluorescence afin de générer une grande image en fluorescence, l'algorithme d'assemblage appliquant l'ensemble de paramètres d'assemblage déterminé lors de l'assemblage des images en lumière visible si la direction d'observation et la perspective des images en lumière visible et des images en fluorescence sont identiques, et l'unité d'assemblage (28) étant conçue pour appliquer l'algorithme d'assemblage en appliquant l'ensemble de paramètres d'assemblage ayant un décalage fixe si la direction d'observation et la perspective des images en lumière visible et des images en fluorescence ne sont pas identiques, le décalage reflétant la relation connue reliant la direction d'observation et la perspective de l'image en fluorescence (7) et de l'image en lumière visible (5),
- une unité d'émission, conçue pour émettre la grande image en lumière visible et la grande image en fluorescence.

10. Le dispositif selon la revendication 9, comprenant en outre :
- une unité de superposition, conçue pour superposer la grande image en lumière visible et la grande image en fluorescence afin de produire une image superposée de la partie corporelle (4), l'unité d'émission étant en outre conçue pour émettre l'image superposée en tant qu'émission de la grande image en lumière visible et de la grande image en fluorescence.

11. Le dispositif (2) selon la revendication 9 ou la revendication 10, dans lequel l'unité (22) de capture d'image en fluorescence et l'unité (24) de capture d'image en lumière visible sont conçues de telle sorte que la direction d'observation, la perspective de l'image en fluorescence et l'image en lumière visible sont identiques, l'unité (22) de capture d'image en fluorescence et l'unité (24) de capture d'image en lumière visible étant en particulier conçues de telle sorte que l'image en fluorescence et l'image en lumière visible sont capturées à travers un seul et même objectif (18).

12. Le dispositif (2) selon l'une quelconque des revendications 9 à 11, dans lequel l'unité (22) de capture d'image en fluorescence et l'unité (24) de capture d'image en lumière visible sont conçues pour capturer simultanément l'image en fluorescence et l'image en lumière visible, en l'absence de commutation temporelle entre un signal de l'image en fluorescence et un signal de l'image en lumière visible.

13. Le dispositif (2) selon l'une quelconque des revendications 9 à 12, dans lequel le dispositif (10) de capture d'image comprend un ensemble de prismes dichroïques conçu pour recevoir la lumière fluorescente et la lumière visible à travers une face d'entrée, comprenant : un premier prisme (P1), un deuxième prisme (P3), un premier prisme compensateur (P2) situé entre le premier prisme (P1) et le deuxième prisme (P3),
un autre ensemble de prismes dichroïques (P5, P6, P7) pour diviser la lumière visible en trois composantes lumineuses, et un deuxième prisme compensateur (P4) situé entre le deuxième prisme (P3) et l'autre ensemble de prismes dichroïques (P5, P6, P7),
le premier prisme (P1) et le deuxième prisme (P3) ayant chacun une section transversale avec au moins cinq angles, chaque angle ayant un angle intérieur d'au moins 90 degrés, les angles du premier prisme (P1) et du deuxième prisme (P3) ayant chacun une face d'entrée (S1, S2) et une face de sortie, et sont chacun conçus de telle sorte qu'un faisceau entrant qui pénètre dans la face d'entrée du prisme respectif dans une direction parallèle à une normale de ladite face d'entrée est réfléchi deux fois à l'intérieur du prisme respectif et sort du prisme respectif par sa face de sortie parallèle à une normale de ladite face de sortie,
la normale de la face d'entrée et la normale de la face de sortie du prisme respectif étant perpendiculaires l'une à l'autre ; et,
lorsque la lumière pénètre dans le premier prisme (P1) à travers la face d'entrée, la lumière est partiellement réfléchie vers la face de sortie du premier prisme (P1), parcourant ainsi une première longueur de trajet entre la face d'entrée du premier prisme (P1) et la face de sortie du premier prisme (P1), et la lumière pénètre partiellement dans le deuxième prisme via le premier prisme compensateur (P2) et est partiellement réfléchie vers la face de sortie du deuxième prisme (P3), parcourant ainsi une deuxième longueur de trajet entre la face d'entrée du premier prisme (P1) et la face de sortie du deuxième prisme (P3),
le premier prisme (P1) étant plus grand que le deuxième prisme (P3) de sorte que les première et deuxième longueurs de trajet sont identiques.

14. Le dispositif (2) selon l'une quelconque des revendications 9 à 13, dans lequel l'unité d'éclairage (16), l'unité (22) de capture d'image en fluorescence et l'unité (24) de capture d'image en lumière visible sont agencées dans un seul dispositif de capture d'images (10), qui comprend en outre une unité de mesure (32) conçue pour mesurer une distance (d) entre la surface (11) de la partie corporelle (4), qui est capturée dans l'image en lumière visible, le dispositif (10) de capture d'image étant en outre, en particulier, conçu pour émettre un signal qui est indicatif de la distance mesurée (d).

15. Un endoscope ou laparoscope conçu en tant que le dispositif (10) de capture d'image dans le dispositif (2) de capture et de traitement d'images selon l'une quelconque des revendications 9 à 14.
